# EUROPÄISCHE PATENTSCHRIFT

(11) **EP 0 857 060 B1**
(45) Veröffentlichungstag und Bekanntmachung des Hinweises auf die Patenterteilung: **30.01.2002**
(21) Anmeldenummer: 96934811.9
(22) Anmeldetag: 24.10.1996
(51) Int. Cl.: A61K 9/14, A61K 47/18, A61K 47/26

(54) **ZUBEREITUNGEN UND VERFAHREN ZUR STABILISIERUNG BIOLOGISCHER MATERIALIEN MITTELS TROCKNUNGSVERFAHREN OHNE EINFRIEREN**
METHOD AND PREPARATIONS FOR STABILIZING BIOLOGICAL MATERIALS BY DRYING METHODS WITHOUT FREEZING
PREPARATIONS ET PROCEDES POUR STABILISER DES MATERIAUX BIOLOGIQUES A L'AIDE DE PROCEDES DE DESSICCATION SANS CONGELATION

(30) Priorität: 25.10.1995 DE 19539574
(43) Veröffentlichungstag der Anmeldung: 12.08.1998
(73) Patentinhaber: Roche Diagnostics GmbH, 68298 Mannheim (DE)
(72) Erfinder: MATTERN, Markus, D-64646 Heppenheim (DE); WINTER, Gerhard, D-69221 Dossenheim (DE)
(74) Vertreter: Braun, Axel
(86) Internationale Anmeldenummer: EP9604627
(87) Internationale Veröffentlichungsnummer: WO9715288

(56) Entgegenhaltungen:
- EP-A- 0 306 824
- EP-A- 0 325 112
- EP-A- 0 444 692
- EP-A- 0 547 422
- WO-A-90/12029
- DE-A- 4 242 863

## Beschreibung

### Technisches Gebiet

Die vorliegende Erfindung betrifft Zubereitungen und Verfahren zur Stabilisierung biologischer Materialien mittels Trocknungsverfahren ohne Einfrieren. Dabei werden gezielt ausgewählte Mischungen von Zuckern und Aminosäuren und deren Derivate sowie von verschiedenen Aminosäuren und deren Derivate beschrieben, mit denen nach Herstellung trockener, teilamorpher Produkte durch Trockenverfahren bei denen nicht eingefroren wird, besonders vorteilhafte Stabilisierung von Peptiden, Proteinen, Glycoproteinen, Antikörpern u.ä. Substanzen erreicht werden kann.

### Stand der Technik

Die Herstellung lagerstabiler (insbesondere bei Raumtemperatur) Zubereitungen von biologisch aktiven und therapeutischen Substanzen wie Peptiden, Proteinen, Glycoproteinen, Nukleotiden, Plasmide, Zellfragmenten, Viren, etc. zu diagnostischen und therapeutischen Zwecken ist heute von großer, ständig wachsender Bedeutung.

Verschiedene Verfahren und Formulierungen zur Herstellung von trockenem biologisch oder therapeutisch aktiven Material sind beschrieben. Unter trockenem Material werden Stoffe und Stoffgemische verstanden, die eine Restfeuchte von höchstens 8% (g/g) bevorzugt höchstens 4% (g/g), besonders bevorzugt höchstens 2% besitzen. Gefriertrocknungsverfahren sind weit verbreitet [F. Franks, Cryo Lett. 11, 93 - 110, (1990); M. J. Pikal, Biopharm. 3 (9), 26 - 30 (1990); M. Hora, Pharm. Research 8(3), 285 - 291 (1992); F. Franks, Jap. J. Freezing Drying 38, 15 - 16, (1992)], weisen aber Nachteile auf. Sie verbrauchen große Energiemengen, erfordern den Einsatz teilweise schädlicher Kältemittel (Frigene), dauern lange Zeit. Für eine Vielzahl von Substanzen, insbes. Proteinen ist der für die Gefriertrocknung notwendige Schritt des Einfrierens schädlich, d.h. destabilisierend. Daher ist dieses Verfahren für manche biologische Materialien gar nicht anwendbar.

Alternativen zur Gefriertrocknung bei der Herstellung trockener Proteinzubereitungen sind Verfahren, die durch Anwendung von Wärme und oder Vakuum das Gut trocknen [F. Franks, R. M. H. Hatley: Stability and Stabilization of Enzymes; Eds. W. J. J. van den Teel, A. Harder, R. M. Butlaar, Elsevier Sci. Publ. 1993, pp. 45 - 54; B. Roser, Biopharm, 4(9), 47 - 53 (1991); J. F. Carpenter, J. H. Crowe, Cryobiol. 25, 459 - 470 (1988)]. Hier sind beispielsweise Vakuumtrocknung mit oder ohne Anwendung von erhöhter Temperatur, Sprühtrocknungsverfahren in verschiedensten Modifikationen inkl. der kombinierten Anwendung von Vakuum und Sprühtechnik sowie Walzentrocknung u.a. Dünnschichttrockenverfahren zu nennen.

In J. F. Carpenter, J. H. Crowe, Biochemistry 28, 3916 - 3922 (1989); K. Tanaka, T. Taluda, K. Miyajima, Chem. Pharm. Bull. 39 (5), 1091 - 94 (1991), DE-C-3520228, EP-B-0229810, WO 91/18091, EP-B-0383569, US 5,290,765, werden Zubereitungen beschrieben, die Zucker oder zuckerartige Stoffe enthalten. Bei der Herstellung trockener Zuckerzubereitungen sind folgende Nachteile, bzw. Probleme bei den im Stand der Technik beschriebenen Verfahren festzustellen: Die Herstellung von wirklich ausreichend trockenen Zuckerzubereitungen ist nicht ohne signifikanten Energie-Einsatz möglich. Dies gilt besonders für Zubereitungen im Endbehältnis. Dabei kommt die Anwendung von Wärme/Hitze in Betracht, die jedoch bezüglich der Stabilität der eingesetzten biologischen Materialien äußerst kritisch zu bewerten ist. Um bei geringer Wärmezufuhr ausreichend Trocknung zu erzielen, sind alternativ drastisch verlängerte Prozeßzeiten oder extrem dünne Schichtdicken anwendbar. Beide Verfahrenswege sind nicht zielführend. Lange Prozeßzeiten sind ökonomisch äußerst ungünstig, außerdem ist das lange Verweilen eines biologischen Wirkstoffes in einer nur langsam an Wasser verarmenden Matrix destabilisierend und somit ebenfalls kritisch. Die Trocknung dünner Schichtdicken führt in vielen Fällen nicht zu der ökonomisch sinnvollen Ausbeute am Produkt, d.h. pro Zeiteinheit und/oder Trocknungsfläche werden nur minimale Produktmengen erhalten. Außerdem ist die Bearbeitung von biologischen Materialien auf sehr großen offenen Trocknungsflächen für die in der pharmazeutischen und diagnostischen Anwendung oftmals erforderliche Sterilität kaum realisierbar.
Trocknungsverfahren, die mittels Vakuum bei niedriger oder geringfügig gegenüber Raumtemperatur erhöhter Temperatur ablaufen, sind schonender. Die Herstellung trockener, lagerstabiler Zuckerzubereitungen ist jedoch in vielen Fällen praktisch kaum durchführbar. Beim Eintrocknen von Zuckerlösungen entstehen zunehmend viskose, zähe Massen. Die in diesen Massen verbliebene Restwassermenge oder Restfeuchte läßt sich nicht in ökonomisch sinnvoller Zeit entfernen, in vielen Fällen kommt die Trocknung auf einem zur Stabilisierung nicht geeigneten, hohen Niveau zum Stillstand. Die Degradation manifestiert sich z. B. in einer Abnahme der Aktivität des gelagerten Materials, in der Bildung von Aggregationsprodukten oder durch das Auftreten von Abbauprodukten mit geringerem Molekulargewicht. Ein zur Stabilisierung von Proteinen etc. geeigneter, niedriger Restwassergehalt läßt sich anhand physikalischer Meßgrößen erkennen. Aus der oben zitierten Literatur geht hervor, daß zur Stabilisierung von Proteinen etc. geeignete Zubereitungen glasartige, das heißt amorphe Struktur aufweisen sollen, deren Glasübergangstemperatur oberhalb der angestrebten Lagertemperatur liegt. Die Glasübergangstemperatur ist diejenige Temperatur, bei der ein amorpher Festkörper aus dem Glaszustand in den zäh-viskosen Zustand übergeht und umgekehrt. Dabei treten drastische Änderungen der Viskosität auf und damit verbunden des Diffusionskoefflzienten und der kinetischen Mobilität der Proteine und anderer Moleküle. Physikalischen Kenngrößen, wie Härte und Modul ändern sich ebenso wie die thermodynamische Zustandsgrößen Volumen, Enthalpie und Entropie. Die Glasübergangstemperatur einer beispielsweise zuckerhaltigen Masse und ihr Restwassergehalt sind physikalisch derart miteinander verknüpft, daß steigende Restwassermengen zu sinkenden Glasübergangstemperaturen führen und umgekehrt. Somit läßt sich aus der Messung der Glasübergangstemperatur z.B. durch Differential Scanning Calorimetrie (DSC), ableiten, ob eine Zubereitung einen zur Stabilisierung geeigneten Restwassergehalt aufweist, bzw. wie oben ausgeführt, ein Trocknungsverfahren erfolgreich ist oder nicht. Neben der Bestimmung der Glasübergangstemperatur mittels DSC läßt sich das Vorhandensein amorpher Strukturen auch mittels Röntgenbeugungsuntersuchungen, optischer und elektronen-mikroskopischer Betrachtungen belegen.

EP-A-0 547 422 betrifft pulverförmige, trockene Vitamin- und/oder Carotinoid-Präparate und Verfahren zu deren Herstellung. Das beschriebene Verfahren schliesst eine thermische Härtung des Pulvers bei Temperaturen von 60 bis 180°C ein.

EP-A-0 444 962 beschreibt Thrombinpräparate die durch Gefriertrocknung vorbereitet werden.

EP-A-0 325 112 offenbart die Stabilisierung von Cephalosporinderivaten. In diesem Dokument scheint die Art der Trocknung den Stabilisierungseffekt nicht zu beeinflussen (Beispiel V), wobei die Beispiele I-IV und VI exclusiv die Gefriertrocknung verwenden.

DE-A-4 242 863 und WO-A-9 012 029 betreffen gefriergetrockene Peptid- oder Proteinformulierungen.

EP-A-0 306 824 betrifft Lösungen und Lyophilisate von Stabilisatoren und Humanproteinen. Verfahren "ohne Einfrieren" oder deren Produkte werden von diesem Dokument nicht erwähnt.

Wünschenswert ist daher die Bereitstellung einer Stabilisierungsmatrix für biologisch oder pharmazeutisch aktive Materialien mit über Lagertemperatur liegender Glasübergangstemperatur, die eine geringe Restfeuchte enthält und Verfahren zur kostengünstigen Herstellung solcher Stabilisierungsmatrizes.

### Beschreibung der Erfindung

Die vorliegende Erfindung befrifft Zubereitungen und Verfahren zur Stabilisierung biologischer Materialien mittels Trocknungsverfahren ohne Einfrieren wie in der Ansprüches.

Überraschenderweise wurde gefunden, dass sich durch Hinzufügen von Zwitterionen mit apolaren Resten zu kohlenhydrathaltigen Massen deren Trocknungsverhalten derart positiv verändern lässt, dass zuvor schlecht trocknende Güter, die dementsprechend keine ausreichend stabilisierenden Eigenschaften aufwiesen, nun sehr schnell zu trocknen waren, ohne Einfrieren der Komponenten, und ausgezeichnete Stabilität der darin formulierten biologisch, insbesondere therapeutisch aktiven Materialien hervorbrachten. Als biologisch, insbesondere therapeutisch aktiven Materialien werden eine oder mehrere Substanzen der Gruppe Proteine, Peptide, Glycoproteine, Lipoproteine, Enzyme, Coenzyme, biologische Membranen, Antikörper, Antikörperfragmente, Zellen, Zellbestandteile, Viren, Virusbestandteile, Vaccine, DNA, RNA, PNA, Plasmide, Vektoren, Pheromone, biologische Therapeutica und Diagnostica und deren Derivate ausgewählt werden.

Weiterhin wurde gefunden, dass überraschenderweise auch kohlenhydratfreie, aus Mischungen bestimmter Zwitterionen bestehende Formulierungen sehr rasch zu trocknen sind und sehr gute stabilisierende Eigenschaften aufweisen. Dabei muss ein Zwitterion mit polarem Rest zusammen mit einem Zwitterion mit apolarem Rest verwendet werden. Solche Zwitterionen sind bevorzugt Aminocarbonsäuren und deren Derivate, besonders bevorzugt pharmazeutisch verträgliche Aminosäuren. Unter Zwitterionen werden niedermolekulare Verbindungen verstanden, deren Molekulargewicht unter 10 kDA, bevorzugt unter 5 kDA liegt. Verfahren werden beschrieben, die es erlauben ohne Anwendung erhöhter Temperatur, d.h. bei Raumtemperatur erfindungsgemässe Zubereitungen so zu trocknen, dass für Zubereitungen zur Stabilisierung von biologisch aktiven insbesondere therapeutisch aktiven Substanzen, die geeigneten Glasübergangstemperaturen erreicht werden. Biologisch aktive Substanzen sind neben therapeutisch wirksamen Substanzen auch solche, die in biotechnologischen Verfahren, wie z. B. Fermentation, verwendet werden. Ebenso solche Substanzen, die z. B. im Pflanzenschutz oder als Insektizide verwendet werden. Unter biologisch aktiven Substanzen werden keine Lebensmittel als solche verstanden.

Die besonderen Vorteile der hier beschriebenen Zubereitungen und Verfahren bestehen darin:
- dass ein Einfrieren während der Trocknung vermieden wird,
- die Durchführung der Trocknung mittels in der chemisch-pharmazeutischen Industrie bereits vorhandenen Gefriertrocknungsanlagen ohne jegliche Umrüstung möglich ist,
- die zur aseptischen Herstellung besonders vorteilhafte Abfüllung in handelsübliche Gefässe, z. B. Glasfläschchen, unverändert beibehalten werden kann,
- Prozesszeiten in der Grössenordnung von Gefriertrocknungsverfahren und weit darunter liegen,
- toxikologisch unbedenkliche Hilfsstoffe eingesetzt werden können
- alle zum Einfrieren erforderlichen Energiemengen eingespart und der Gebrauch von umweltschädlichen Kältemitteln drastisch reduziert werden kann,
- die erhaltenen Produkte gut sichtbar, rasch sich wieder auflösende "Kuchen" darstellen,
- durch das rasche Erreichen des teilamorphen Zustandes, das biologische Material weniger degradiert als durch die im Stand der Technik beschriebenen Verfahren.

Es ist festzustellen, dass die besonderen Vorteile der hier dargestellten Rezepturen aus bestimmten Mischungen von Zuckern und Aminosäuren, sowie von bestimmten Mischungen von mindestens 2 Aminosäuren auch wirksam werden, wenn sie im Rahmen anderer Trocknungsverfahren, die das Einfrieren vermeiden, eingesetzt werden. Auch bei Sprühtrocknung, Walzentrocknung etc. kommt die trocknungsbeschleunigte Wirkung der Zusätze sowie die Eigenschaft der Zubereitungen zur Ausbildung amorpher oder teilamorpher Systeme voll zu Tage.

Es ist wesentlich, daß durch DSC und/oder Röntgenstrukturanalyse oder andere geeignete Verfahren nachweisbar signifikante amorphe Anteile vorhanden sind und die Zubereitung nicht vollständig kristallinen Charakter hat. Kristalline Zubereitungen sind nicht geeignet um ausreichende Stabilität bei empfindlichen biologischen Substanzen zu erzielen. Vollständig amorphe Zubereitungen sind zur Stabilisierung geeignet und somit prinzipiell erfindungsgemäß, besonders jedoch teilamorphen Zubereitungen.

### Beschreibung der Abbildungen

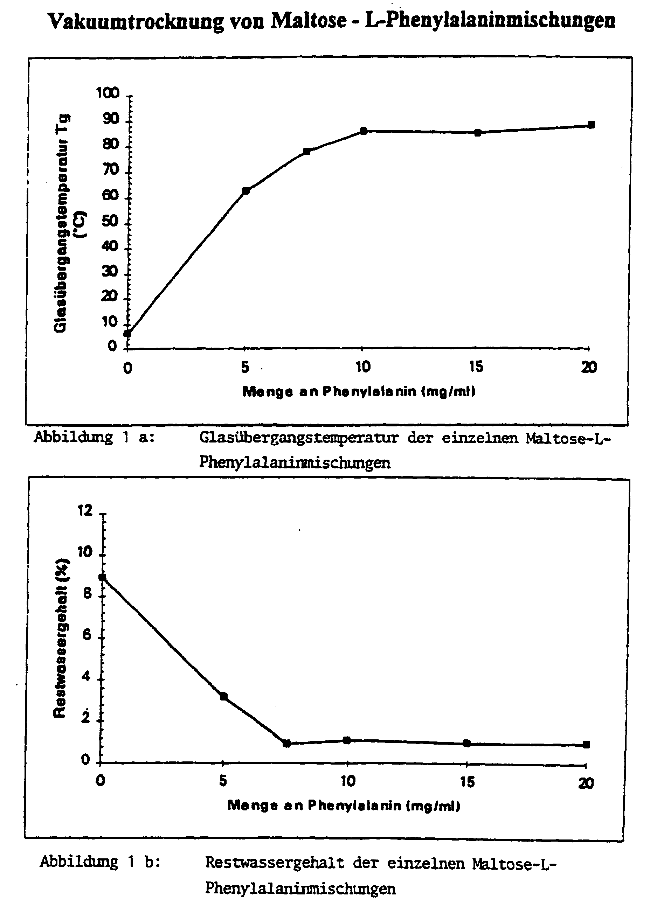

- Abb. 1 a:: Glasübergangstemperaturen der einzelnen Maltose-L-Phenylalaninmischungen
- Abb. 1 b:: Restwassergehalt der einzelnen Maltose-L-Phenylalaninmischungen
- Abb. 2:: Pulverdiffraktogramme von vakuumgetrockneten
(a) Phenylalanin (Wasergehalt 1.2%/kristallin),
(b) Maltose (Wassergehalt 4.0%, Tg=50°C) und
(c) Phenylalanin und Maltose in erfindungsgemäßer Weise zubereitet (Wassergehalt 0.7%, Tg=88°C).
Die Diffraktogramme wurden mit einem konventionellen Gerät (Phillips 1730 X-ray) und dazugehöriger Software aufgenommen. Die Meßtemperatur beträgt 25°C, die Winkelauflösung (2θ) 0. 05°. Meßbedingungen: 1 s pro Winkel bei 40 kV Röhrenspannung und 40 mA Stromstärke.

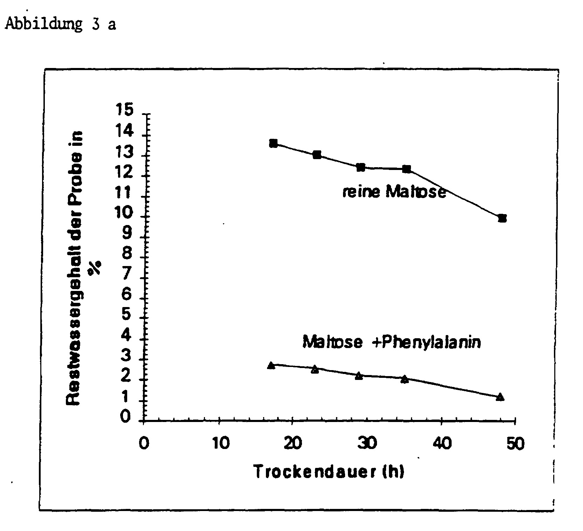

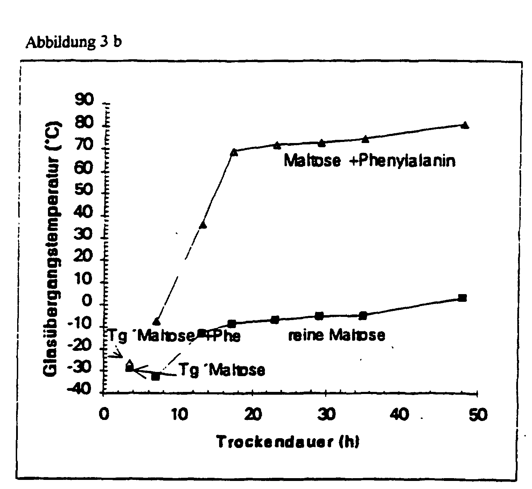

- Abb. 3:: Zeitlicher Verlauf des
(a) Restwassergehalts und der
(b) Glasübergangstemperatur einer erfindungsgemäßen Maltose/Phenylalaninzubereitung.

### Detaillierte Beschreibung der Erfindung

In 13 Beispielen und 10 Vergleichsbeispielen ist die Erfindung exemplarisch dargelegt, und wird nachfolgend erläutert. Dabei wurden Formulierungen und Verfahren gefunden, die die Trocknung zuckerhaltiger Massen mittels Vakuumtrocknung drastisch verbessern und beschleunigen und zur Stabilisierung relevanter therapeutischer und diagnostischer biologischer Materialien geeignet sind. Des weiteren werden völlig neuartige Zusammensetzungen aufgezeigt, die den Zweck der Stabilisierung unter Beibehalt der optimierten Trocknungscharakteristik erfüllen.

Diese Zusammensetzungen enthalten bevorzugt entweder mindestens ein Zwitterion mit apolarem Rest (z. B. eine Aminosäure wie Phenylalanin) und Zucker, wobei die Glasübergangstemperatur des Zuckers durch diesen Zwitterionzusatz deutlich erhöht wird. Alternativ können auch Gemische von verschiedenen speziell ausgewählten Aminosäuren oder deren Derivate verwendet werden. Diese Gemische setzen sich aus einem Zwitterion mit apolarem und einem Zwitterion mit polarem Rest zusammen. Zu diesen Gemischen können auch noch Zucker zugesetzt werden.

Als Arbeitshypothese wurde gefunden, daß besonders Mischungen zwischen einem Zucker oder polar zwitterionischen Substanz (z. B. Arginin, Asparaginsäure, Glutaminsäure, Histidin, Citrullin, Lysin) und apolaren zwitterionischen Substanz (z. B. Phenylalanin, Isolencin, Methionin, Valin, Alanin, Glycin, Tryptophan, Cystein) oder deren Derivate (z. B. Acetylphenylalaninethylester) die erfindungsgemäß gewünschten Resultate ergibt. Es ist leicht möglich sowohl das Verfahren abzuwandeln, wie die anhand der Beispiele beschriebenen Stofflisten zu erweitem.

Besonders bevorzugte biologisch oder therapeutisch aktiven Materialien sind Antikörper (monoklonal oder polyklonal), Enzyme und Humanproteine bzw. Humanpeptide, wie z. B. rekombinantes humanes Erythropoietin (rh-EPO), rekombinanter humaner Granulocyten Colony Stimulating Factor (rh-G-CSF) oder rekombinanter Plasminogen Aktivator (rPA), nGF, PTH, Ularitide, Plasmide, Viren, GUP, BP-5.

An wirkstoffreien Zubereitungen wurde erarbeitet, in welcher Weise der Zusatz von Aminosäuren die Trocknung von Zuckermatrices verändert. In Beispiel 1 ist dargestellt, daß der Zusatz von Phenylalanin und Arginin zu Maltose deren Trockenverhalten in Abhängigkeit von der zugesetzten Menge dieser Zuschlagstoffe verbessert. Die Glasübergangstemperatur läßt sich durch gezielten Zusatz dieser Hilfsstoffe bei gleichen Trocknungsbedingungen um über 65 K erhöhen, der korrespondierende Restwassergehalt läßt sich leicht auf unter 1 % absenken. Aus Beispiel 1 geht hervor, daß das dabei angewandte Verfahren innerhalb von 48 h ohne jegliche Wärmeeinwirkung zum gewünschten Ergebnis führt. Maltose ohne die erfindungsgemäß zugesetzten Hilfsstoffe weist unter diesen Bedingungen einen Restwassergehalt von 7-8 % auf, die Glasübergangstemperatur (Tg) liegt unterhalb der Raumtemperatur und somit ist dieses System nicht zur Stabilisierung von Proteinen etc. geeignet.

Die Herstellung erfindungsgemäßer Zubereitungen aus Saccharose und einer Aminosäure aus der Gruppe der erfindungsgemäß zur Herstellung stabilisierender, teilamorpher Produkte geeigneten Aminosäuren vermag bestimmte Nachteile bei der Formulierung mit Saccharose zu vermeiden, während die der Saccharose eigenen Vorteile voll zum Tragen kommen können. Saccharose hat im Vergleich zu anderen, in der Literatur erwähnte Zuckern eine relativ niedrigere Glasübergangstemperatur bei entsprechend normierten Wassergehalten. Es ist deshalb bei der Herstellung saccharosehaltiger, trockener Zubereitungen besonders schwierig, hohe Tgs zu erreichen, die deutlich oberhalb der angestrebten Lagertemperatur liegen. Weiterhin ist es schwierig Saccharose durch Verdunstungstrocknung überhaupt in eine amorphe Form zu überführen, der Zucker kristallisiert leicht aus und bildet somit leicht die zur Stabilisierung biologische Wirkstoffe ungünstige kristalline Struktur. Außerdem kann beobachtet werden, daß amorphe Saccharosemassen im Laufe der Lagerung vergleichsweise rasch in großem Ausmaß Kristalle bilden und nach Ablauf bestimmter Lagerzeiten vollständig kristallisieren können. Auch bei diesem Vorgang verliert eine derartige Zubereitung ihre stabilisierenden Eigenschaften. Alle diese mit der Anwendung von Saccharose verknüpften Probleme, Risiken und Unzulänglichkeiten können durch den erfindungsgemäßen Zusatz von Aminosäuren der entsprechende Gruppe behoben werden. Besonders bevorzugt ist dabei die Anwendung von Phenylalanin und Arginin (Beispiel 2). In Vergleichsbeispiel A wird gezeigt, daß auch unter Anwendung längerer Trocknungszeiten und erhöhter Temperaturen reine Zuckermassen sich nicht effizient trocknen lassen. Der trocknungsverbessernde Effekt von Aminosäuren und Zucker läßt sich sowohl mit einzelnen Aminosäuren als auch mit Aminosäuremischungen erreichen. Beispiele 3 und 4 liefern dazu entsprechende Ergebnisse an Maltose und Saccharose-Systemen. Es sind auch Aminosäuren gefunden worden, die keine trocknungsverbessernden Effekte haben, z. B. Histidin (Vergleichsbeispiel B). In Beispiel 5 wird gezeigt, daß neben Aminosäuren auch deren strukturverwandte Derivate trocknungsverbessernde Effekte aufweisen können. Die Auswahl bestimmter Aminosäuren wird ausführlich, wenn auch nicht limitierend oder völlig umfassend in Beispiel 6 beschrieben. Es ist festzuhalten, daß bei weitem nicht jede Aminosäure den gewünschten Effekt herbeiführt, sondern nur bestimmte Aminosäuren. Auch das Ausmaß der Effekte ist unterschiedlich, so daß besonders bevorzugte Kombinationen oder Zubereitungen benannt werden können. Diese sind vor allem Phenylalanin, Tryptophan, Leucin und Isoleucin. Aus Beispiel 1 und 6 läßt sich weiterhin ableiten, daß die Mischung von Aminosäuren unter Beibehaltung des trocknungsverbessernden Effektes möglich ist. Arginin allein zeigt keinen positiven Effekt, wohl aber in Mischung mit Phenylalanin.

Das Verhalten von Aminosäuren bei der Vakuumtrocknung wurde untersucht, um festzustellen, ob auch ohne Zuckermatrix mittels Aminosäuren Zubereitungen erhalten werden können, die eine Glasübergangstemperatur oberhalb der Raumtemperatur aufweisen. Überraschenderweise wurde gefunden, daß eine reine Aminosäure alleine nur kristalline Strukturen ausbildet, während bestimmte Aminosäuresalze und Mischungen von Aminosäuren glasartige Matrizes bilden (Vergleichsbeispiel C und Beispiel 7). Zur Herstellung von amorphen Strukturen ist es erforderlich, gezielt verschieden Aminosäuren auszuwählen. Überraschenderweise wurde gefunden, daß sich Aminosäuren in 2 Gruppen einteilen lassen, die offensichtlich unterschiedliche Eigenschaften aufweisen. Es ist notwendig aus jeder Gruppe mindestens eine Aminosäure auszuwählen und eine entsprechende Mischung herzustellen und diese zu trocknen. Wie bei der Formulierung von Zucker-Arninosäure-Mischungen, ist auch hier ein bestimmtes Mischungsverhältnis erforderlich um erfindungsgemäße Zubereitungen zu erhalten (Beispiel 7). Dann erhält man eine Matrix mit amorphen Anteilen, die zur Stabilisierung biologischer Wirkstoffe geeignet ist.

Die Wirksamkeit der verbesserten Trocknung bezüglich des eigentlichen Zieles, der Stabilisierung von biologisch aktivem Material, exemplarisch gezeigt an Proteinen, wird ausführlich in den Beispielen 8 - 12 und den Vergleichsbeispielen D - J belegt. Beispiele 8 und 9 mit den Vergleichsbeispielen D - G beschreiben die Stabilisierung von rh-G-CSF, Beispiel 10 und Vergleichsbeispiel H von Erythropoietin und Beispiele 11 und 12 sowie Vergleichsbeispiele I und J die Stabilisierung von Lactat-Dehydrogenase. Anhand von Lagerzeiten von einem Protein (rh-G-CSF, Beispiele 8 und 9 bzw. Vergleichsbeispiele D, E und F, G), einem Glykoprotein (rh-EPO, Beispiel 10 und Vergleichsbeispiel H) und einem Enzym (LDH, Beispiele 11, 12 und Vergleichsbeispiel I und J) wird beispielhaft die überraschend stark verbesserte Lagerstabilität der erfindungsgemäßen Zubereitungen gegenüber vakuumgetrockneten Zubereitungen ohne Hilfsstoffe und weiteren Zubereitungen gezeigt. Die Veränderungen an verschiedenen rh-G-CSF-Zubereitungen unter Lagerbedingungen bei verschiedenen Temperaturen wird in den Beispielen dargestellt. Nur erfindungsgemäße, d.h. teilamorphe, glasartige Zubereitungen zeigen nach 6 Monaten keine nennenswerte Degradation im Lager-Temperaturbereich von wenigen Grad Celsius (Kühlschrank) bis zu 40 °C (Beispiele 8 und 9). Entsprechende hilfsstoffreie vakuumgetrocknete Zubereitungen (Vergleichsbeispiel D) zeigen bei Raumtemperatur und erhöhter Lagertemperatur (40 °C) eine deutliche Monomerenabnahme von bis zu 20 %. Nicht-amorphe, sondern zäh-viskose Zubereitungen, die oberhalb der Glasübergangstemperatur gelagert werden, zeigen schon bei RT nach 5 Wochen signifikante Abnahmen der Monomerenkonzentration (Vergleichsbeispiele D + E). Kristalline Zubereitungen (Vergleichsbeispiel G und J) zeigen ebenso signifikant verkürzte Lagerzeiten. Aus einem Vergleich von Beispiel 8 und Vergleichsbeispiel E erkennt man, daß bei erhöhter Lagertemperatur (40 °C) der Zusatz von Aminosäuren zu Maltose als Stabilisatoren die Lagerzeit, bei der weniger als 10 % der Monomeren von G-CSF aggregieren, mehr als verzehnfacht wird. Ein Vergleich von Beispiel 9 mit Vergleichsbeispiel G zeigt, daß auch die Wahl der Aminosäuren entscheidend für die stark verlängerte Lagerzeit ist. Ein Vergleich des Monomerenanteils bei dem Glycoprotein EPO (Beispiel 10, Vergleichsbeispiel H) zeigt, daß erfindungsgemäße Zubereitungen bei Raumtemperatur und erhöhter Lagertemperatur gegenüber vakuumgetrocknetem EPO ohne Hilfsstoffe deutlich überlegen sind. Bei 5wöchiger Lagerung des empfindlichen Enzyms LDH als erfindungsgemäße Zubereitungen (Beispiele 11 und 12) im Vergleich zum vakuumgetrockneten LDH ohne Hilfsstoffe (Vergleichsbeispiel I) und kristalliner Zubereitung (Vergleichsbeispiel J) zeigt sich, daß nur erfindungsgemäße Zubereitungen bei Raumtemperatur oder höheren Lagertemperaturen (30°C) ohne drastischen Aktivitätsverlust gelagert werden können, Beachtenswert ist dabei auch die zusätzliche Stabilisierung des Enzyms durch die erfindungsgemäße Zubereitung direkt nach der Präparation der Proben (Aktivität bei 0 Wochen > 80 % vs 65 % bei hilfsstoffreier Zubereitung bzw. 10% bei krist. Zubereitung). Ein typischer zeitlicher Verlauf des Trockungsvorgangs einer erfindungsgemäßen Mischung ist in Beispiel 13 exemplarisch gezeigt.
Zur Herstellung erfindungsgemäßer Mischungen mindestens zweier Aminosäuren zur Erzielung schnelltrocknender, glasartiger Zubereitungen sind jeweils mindestens eine Aminosäure und deren Derivate aus den nachfolgend genannten 2 Gruppen auszuwählen:
- Gruppe 1:: Arginin, Asparaginsäure, Glutaminsäure, Histidin, Citrullin, Lysin, Ornithin
- Gruppe 2:: Phenylalanin, Isoleucin, Leucin, Methionin, Valin, Alanin, Glycin, Tryptophan, Acetylphenylalaninethylester, Cystein, Sarcosin

Die alleinige Anwendung nur einer Aminosäure oder mehrerer Aminosäuren aus nur einer der beiden Gruppen führt nicht zu den erfindungsgemäß vorteilhaften Zubereitungen. Erfindungsgemäße Stoffgemische können, wie exemplarisch gezeigt, aufgefunden werden, indem einer Lösung eines Stabilisators biologisch oder therapeutisch aktiver Substanzen ein Zwitterion mit apolarem Rest, z. B. Phenylalanin oder dessen Derivate in verschiedenen Mengen zugemischt wird. Die bei Raumtemperatur getrockneten Gemische werden anschließend mittels DSC überprüft, ob sie eine durch den zwitterionischen Zusatz erhöhte Glasübergangstemperatur aufweisen. Die Glasübergangstemperatur hat sich dabei gegenüber Zubereitungen ohne erfindungsgemäße Zusätze um 10K, bevorzugt um 20K, besonders bevorzugt um 40K erhöht. Die erfindungsgemäßen vorteilhaften Zubereitungen sind teilamorph, besitzen eine Glasübergangstemperatur über 4°C, bevorzugt über 20°C, besonders bevorzugt über 40°C und haben entsprechende Restfeuchten von weniger als 6%, bevorzugt weniger als 4%. Ihre scheinbare Dichte entsprechend der Schüttdichte ist zumindest 10%, bevorzugt 50% höher als die der entsprechenden Lypophilisate. Sie behalten ihre spröde, glasartige, kompakte, teilamorphe Struktur über mindestens 2 Wochen, bevorzugt 2 Monate, besonders bevorzugt 1 Jahr. Außerdem ist ihre Trocknungszeit, (d.h. der Zeitpunkt an dem die gleiche Restfeuchte erreicht wird), gegenüber Stoffinischungen, die nur ein Kohlenhydrat oder Zwitterion mit apolarem Rest enthalten, vorzugsweise um 25% verringert, besonders bevorzugt halbiert oder sogar geviertelt. Diese Substanzgemische können auch gemahlen oder anderweitig weiterverarbeitet werden, z. B. in therapeutischen Mitteln oder Diagnostika in Kombination mit üblichen Hilfs- und Trägerstoffen verwendet werden. Therapeutische Mittel sind therapeutische Zubereitungen, die ein oder mehrere therapeutisch wirksame Agens neben üblichen Hilfs- und Zusatzstoffen enthalten. Sie können in Form von Tabletten, Kapseln oder Feststoffen, aus denen durch Zugabe von Flüssigkeit (z.B. steriles Wasser oder Puffer) therapeutisch wirksame Lösungen (z. B. Infusionslösungen) hergestellt werden, vorliegen. Sie sind weiterhin besonders geeignet, um mittels verschiedener Verfahren als feste Substanz appliziert zu werden, z. B. als Nasenspray, Inhalat oder transdermales Pulver usw.

### Beispiel 1:

### Vakuumtrocknung von Maltose-L-Arginin-L-Phenylalaninmischungen

Es wurde eine Lösung mit einem Gehalt von 50 mg Maltosemonohydrat und 0,1 mg Polysorbat 80 pro ml hergestellt. Dieser wurden dann steigende Mengen an L-Arginin und L-Phenylalanin zu gleichen Anteilen (g/g) zugefügt. Die so hergestellten Lösungen wurden sterilfiltriert (0,22 µm Cellulosenitratfilter) und dann jeweils 1 ml der Lösung in 2ml-Vials gefüllt und Gefriertrocknungsstopfen aufgesetzt. Die so vorbereiteten Proben wurden in gleicher Weise bei 20°C unter vermindertem Druck 48 h lang vakuumgetrocknet. Nach der Trocknung wurde der Wassergehalt der Proben nach Karl-Fischer bestimmt und die Glasübergangstemperatur mittels Differentialthermoanalyse (Perkin Elmer DSC7 - Aufheizrate der Proben = 10 K/min) ermittelt. Die Meßergebnisse zeigen, daß sich das Trockenverhalten der Maltose durch Zugabe bestimmter Mengen der Aminosäuren deutlich ändert. Ab 7,5 mg jeder Aminosäure nimmt der Wassergehalt der Proben deutlich ab und die Glasübergangstemperatur steigt dementsprechend an. Bei je 10 mg L-Arginin und L-Phenylalanin sind Werte erreicht, die sich durch weitere Erhöhung der Aminosäureanteile im trockenen Produkt nicht mehr steigern lassen. Zu der Zuckerlösung mit 50 mg Maltosemonohydrat und 0,1 mg Polysorbat 80 pro ml wurden steigende Mengen an Aminosäuren gegeben. Die nach der Trocknung resultierenden Produkte wiesen die hier angegebenen Wassergehalte und Glasübergangstemperaturen auf.

**Tabelle 1:**

| **L-Arginin [mg/ml]** | **L-Phenylalanin [mg/ml]** | **Restwassergehalt [%]** | **Glasübergangstemperatur [°C]** |
|---|---|---|---|
| 0 | 0 | 7,68 | 12,86 |
| 1 | 1 | 7,95 | 12,47 |
| 2,5 | 2,5 | 7,71 | 12,91 |
| 5 | 5 | 7,75 | 13,67 |
| 7,5 | 7,5 | 3,55 | 52,04 |
| 10 | 10 | 0,54 | 80,57 |
| 12,5 | 12,5 | 0,76 | 73,14 |

### Beispiel 2:

### Vakuumtrocknung von Saccharose - L-Arginin-L-Phenylalaninmischungen

Zu einer Saccharoselösung, die 50 mg Saccharose und 0,1 mg Polysorbat 80 pro ml enthielt, wurden steigende Mengen L-Arginin und L-Phenylalanin zu gleichen Anteilen (g/g) zugefügt. Die Proben wurden wie bei Beispiel 1 hergestellt, getrocknet und analysiert. Bei Mengen bis je 10 mg Aminosäure erhielt man vollkommen kristalline Produkte. Erst ab 10 mg L-Arginin und 10 mg L-Phenylalanin pro ml waren teilamorphe Produkte mit einem Glasübergang zu identifizieren. In diesem Beispiel wurde durch Zugabe von Aminosäuren nicht nur das Trockenverhalten einer Lösung verbessert, sondern das System durch diese Zugabe in den teilamorphen Zustand überführt. Zu der Zuckerlösung mit 50 mg Saccharose und 0,1 mg Polysorbat 80 pro ml wurden steigende Mengen an Aminosäuren gegeben. Die nach der Trocknung resultierenden Produkte wiesen die hier angegebenen Wassergehalte und Glasübergangstemperaturen auf.

**Tabelle 2:**

| **L-Arginin [mg/ml]** | **L-Phenylalanin [mg/ml]** | **Restwassergehalt [%]** | **Glasübergangstemperatur [°C]** |
|---|---|---|---|
| 0 | 0 | 2,97 | **kristallin** |
| 1 | 1 | 1,11 | **kristallin** |
| 2,5 | 2,5 | 3,46 | **kristallin** |
| 5 | 5 | 6,11 | **kristallin** |
| 10 | 10 | 3,43 | 18,56 |
| 15 | 15 | 1,53 | 53,70 |
| 20 | 20 | 1,60 | 58,78 |

### Vergleichsbeispiel A

### Vakuumtrocknung reiner Zuckerlösungen

Es wurden eine Maltosemonohydrat- und eine Saccharoselösung mit der Konzentration 50 mg/ml hergestellt. Diese Zuckerlösungen wurden dann filtriert, abgefüllt und analysiert wie in Beispiel 1 beschrieben. Es konnte gezeigt werden, daß es selbst bei einer 72 stündigen Trocknung bei 50°C unter vermindertem Druck nicht möglich ist, 50 mg Zucker in einem 2ml Vial auf eine befriedigende Restfeuchte zu trocknen, so daß der Glasübergang oberhalb 25°C liegt. Das Maltoseprodukt wies zähe Konsistenz auf und hatten einen Restwassergehalt von 6,4%. Der Glasübergang lag bei 20°C. Bei der Saccharose waren noch 6,0% Restwasser vorhanden, der Glasübergang lag bei 14°C. Zum Vergleich wurden die reinen Zuckerlösungen auch 48 h bei 20°C unter vermindertem Druck getrocknet. Die resultierenden Produkte waren noch feuchter und der Glasübergang lag deshalb noch tiefer, als bei den bei 50°C getrockneten Proben. Dieser Versuch zeigt deutlich, daß es nur durch Zugabe von bestimmten Aminosäuren möglich ist, Zuckerschichten in Injektionsfläschchen oder ähnlichen Behältnissen mittels Vakkumtrocknung auf geringe Restfeuchten zu trocknen. Das verbesserte Trockenverhalten durch Zugabe der Aminosäuren wird so deutlich.

**Tabelle 3:**

| | **Maltose** | | **Saccharose** | |
|---|---|---|---|---|
| **Trocknung** | **Restwassergehalt** | **Glasübergangstemperatur** | **Restwassergehalt** | **Glasübergangstemperatur** |
| 72 h bei 50°C | 6,4 % | 20,0°C | 6,0 % | 14,0°C |
| 48 h bei 20°C | 8,9 % | 6,1°C | 9,3 % | - 1,8°C |

### Beispiel 3:

### Vakuumtrocknung von Maltose -L-Phenylalanin und Maltose-L-Isoleucingemischen

In diesem Versuch wurden nun binäre Mischungen von Aminosäuren und Maltosemonohydrat hergestellt. Er soll überprüfen, ob die hier eingesetzten Aminosäuren trocknungsverbessernde Eigenschaften besitzen und welche Mengenabhängigkeit des tracknungsverbessernden Effekts bei den einzelnen Aminosäuren besteht. Zu einer Lösung, die 50 mg Maltosemonohydrat pro ml enthielt, wurden steigende Mengen an L-Phenylalanin oder L-Isoleucin hinzugegeben. Die so hergestellten Lösungen wurden sterilfiltriert ( 0,22 µm Cellulosenitratfilter ) und dann jeweils 1 ml der Lösung in 2ml-Vials gefüllt und Gefriertrocknungsstopfen aufgesetzt. Die so vorbereiteten Proben wurden bei 20°C unter vermindertem Druck 48 h lang vakuumgetrocknet. Nach der Trocknung wurde der Wassergehalt der Proben nach Karl-Fischer jeweils 4fach bestimmt und die Glasübergangstemperatur mittels Differentialthermoanalyse (Perkin Elmer DSC7 - Aufheizrate der Proben = 10 K/min) bei je zwei Proben pro Mischung ermittelt.

### a. Ergebnis Maltose - L-Phenylalanin

Die Meßergebnisse zeigen deutlich den positiven Einfluß des L-Phenylalanins auf das Trockenverhalten von Maltose. Bereits geringe Mengen an L-Phenylalanin reichen aus, um die Glasübergangstemperatur eines Zuckerglases bei gleichbleibenden Trockenbedingungen um ca. 50°C zu steigern (Abb. 1 a und 1 b). Bei 10 mg/ml L-Phenylalanin erreicht der trocknungsverbessernde Effekt ein Maximum. Durch Zugabe von größeren Mengen an L-Phenylalanin ist keine Verbesserung mehr möglich. Die Glasübergangstemperatur wurde so durch den Zusatz von L-Phenylalanin gegenüber der reinen Maltose um ca. 80 °C gesteigert. Bei großen Mengen L-Phenylalanin (10-20 mg/ml) kann man in diesem Versuch keine Unterschiede bezüglich des Trockenverhaltens erkennen. Dies ändert sich aber bei verkürzten Trockenzeiten. Hier ist mit steigenden L-Phenylalaninmengen ein Ansteigen der Glasübergänge auch im Bereich 10 - 20 mg L-Phenylalanin pro ml zu sehen. Die Tabelle 4 zeigt die Menge an L-Phenylalanin in der Zuckerlösung und den daraus resultierenden Restwassergehalt und die Glasübergangstemperatur Tg.

**Tabelle 4:**

| **L-Phenylalanin [mg/ml]** | **Restwassergehalt [%]** | **Glasübergangstemperatur [°C]** |
|---|---|---|
| 0 | 8,91 | 6,1 |
| 5 | 3,21 | 62,8 |
| 7,5 | 0,95 | 77,7 |
| 10 | 1,12 | 86,0 |
| 15 | 0,99 | 85,2 |
| 20 | 0,99 | 88,2 |

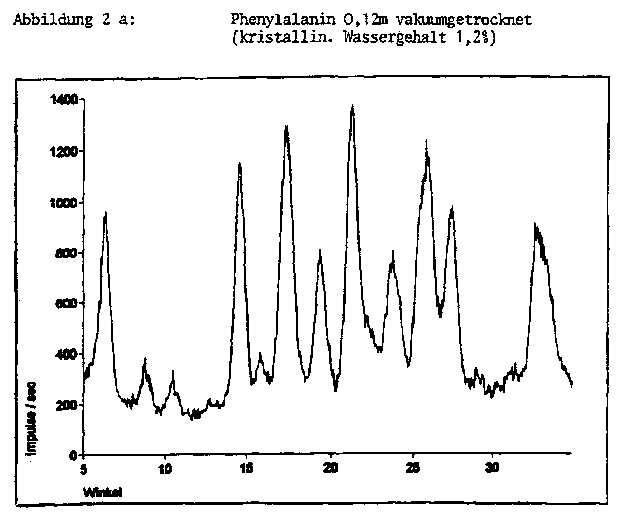

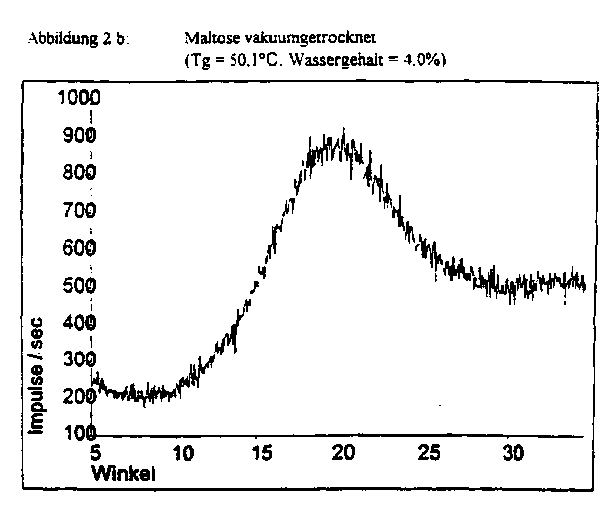

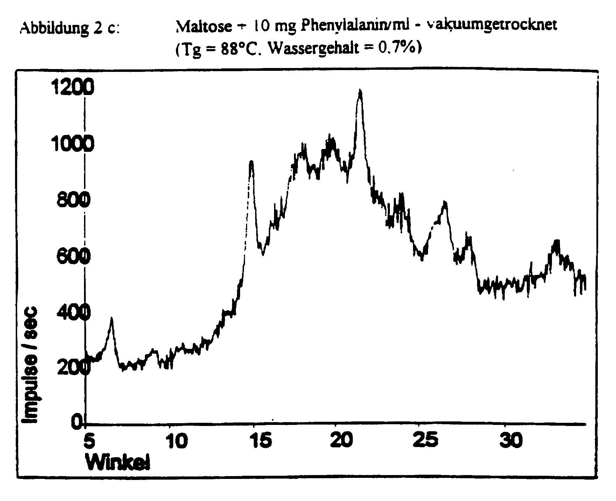

Außerdem wurden Pulverdiffraktogramme von vakuumgetrockneten Phenylalanin, Maltose und einer erfindungsgemäßen Mischung von Phenylalanin und Maltose aufgenommen (Abb. 2a, 2b, 2c). Das reine Phenylalanin zeigt ein typisches Diffraktogramm einer kristallinen Substanz (Abb. 2a), während Maltose das Diffraktogramm einer amorphen Substanz zeigt (Abb. 2b). Nur bei der erfindungsgemäßen Mischung kommt es zur Ausbildung teilamorpher Strukturen, erkennbar an den diskreten Beugungsmaxima auf einem breiten Hintergrundssignal (Abb. 2c).

### b. Ergebnis Maltose -L-Isoleucin

Der Stammlösung mit 50 mg Maltosemonohydrat pro ml wurden verschiedene Mengen L-Isoleucin zugegeben, und die einzelnen Mischungen wurden bei 20°C getrocknet. Mit steigender Aminosäuremenge wird ein trocknungsverbessernder Effekt deutlich. Mit einer Zugabe von 20 mg/ml L-Isoleucin (Mischungsverhältnis 5:2 nach Gewicht) läßt sich der Tg eines Maltoseproduktes um ca. 20°C steigern. Die Tabelle 5 zeigt die Menge an L-Isoleucin in der Zuckerlösung und den daraus resultierenden Restwassergehalt und die Glasübergangstemperatur Tg.

**Tabelle 5:**

| **Isoleucin [mg/ml]** | **Restwassergehalt [%]** | **Glasübergangstemperatur [°C]** |
|---|---|---|
| 0 | 8,91 | 6,1 |
| 5 | 7,84 | 13,7 |
| 10 | 7,52 | 17,7 |
| 15 | 6,77 | 19,4 |
| 20 | 5,34 | 24,7 |

### Beispiel 4:

### Vakuumtrocknung von Saccharose-L-Leucin

In folgenden Versuchen wurden binäre Mischungen von Saccharose mit verschiedenen Aminosäuren hergestellt. Es sollte überprüft werden, ob die eingesetzten Aminosäuren auf die Saccharose einen trocknungsverbessernden Effekt ausüben. Zu einer Lösung die 50 mg Saccharose pro ml enthielt wurden steigende Mengen an L-Leucin zugegeben. Die Lösungen wurden wie in Beispiel 3 beschrieben behandelt.

### Ergebnis Saccharose - L-Leucin

Saccharose bildet mit geringen Mengen L-Leucin ein kristallines Produkt. Diese Kristallbildung konnte auch schon in Beispiel 2 mit L-Arginin und L-Phenylalanin beobachtet werden. Saccharose bildet also bei der Mischung mit bestimmten Aminosäuren kristalline Produkte. Der reine Zucker und Mischungen mit größeren Anteilen an L-Leucin bilden Systeme mit einem Glasübergang. Dies bedeutet, daß ein teilamorphes Gerüst vorliegt. So wird deutlich, daß erst Konzentrationen ab 15 mg/ml L-Leucin die Trocknungseigenschaften der reinen Saccharose verbessern und der Glasübergang durch Zugabe dieser Aminosäure um ca. 18 °C gesteigert werden kann. L-Leucin ist somit eine Aminosäure mit einem trocknungsverbessernden Effekt. Die Tabelle 6 zeigt die Menge an L-Leucin in der Zuckerlösung und den daraus resultierenden Restwassergehalt und die Glasübergangstemperatur Tg der Endprodukte.

**Tabelle 6:**

| **L-Leucin [mg/ml]** | **Restwassergehalt [%]** | **Glasübergangstemperatur [°C]** |
|---|---|---|
| 0 | 9,34 | - 1,8 |
| 5 | 6,23 | kristallin |
| 10 | 6,50 | kristallin |
| 15 | 5,81 | 16,4 |
| 20 | 5,02 | 16,1 |

### Vergleichsbeispiel B

### Vakuumtrocknung von Saccharose-L-Histidin-Gemischen

Die Versuche wurden durchgeführt wie in Beispiel 4 angegeben. Statt L-Leucin wurde L-Histidin verwendet. Die Mischung Saccharose - L-Histidin bildet bei der Vakuumtrocknung amorphe Produkte, bei denen kein trocknungsverbessernder Effekt zu beobachten ist. Unabhängig vom Mischungsverhältnis trocknen die Gerüste schlecht und die Restwassergehalte und Glasübergänge der Mischungen liegen in der gleichen Größenordnung wie die Ergebnisse des reinen Zuckers. Folglich hat L-Histidin keinen trocknungsverbessernden Effekt. Die Tabelle 7 zeigt die Menge an L-Histidin in der Zuckerlösung und den daraus resultierenden Restwassergehalt und die Glasübergangstemperatur Tg der Endprodukte.

**Tabelle 7:**

| **L-Histidin [mg/ml]** | **Restwassergehalt [%]** | **Glasübergangstemperatur [°C]** |
|---|---|---|
| 0 | 9,34 | - 1,8 |
| 5 | 11,23 | -1,4 |
| 20 | 9,78 | -2,6 |

### Beispiel 5:

### Vakuumtrocknung von Saccharose - L-Tryptophan und Saccharose - N-acetyl-L-phenylalaninethylester (APE) Gemischen

In diesem Versuch wurde eine Lösung mit 10 mg L-Tryptophan pro ml und eine Lösung mit 3 mg APE pro ml hergestellt (APE hat nur eine begrenzte Löslichkeit in Wasser). Beiden Lösungen wurde in steigenden Mengen Saccharose zugegeben. Die so erhaltenen Lösungen wurden wie in Beispiel 3 beschrieben behandelt und getrocknet. Zum Vergleich wurde bei diesem Versuch unter gleichen Trockenbedingungen eine Saccharoselösung (50 mg/ml) mit getrocknet. Diese wies im Endprodukt einen Restwassergehalt von 9,98% und einen Glasübergang von -6,25°C auf.
a. Die Tabelle 8 zeigt die Menge an Saccharose in der L-Tryptophanlösung (10mg/ml) und den daraus resultierenden Restwassergehalt und die Glas übergangstemperatur der Endprodukte.

**Tabelle 8:**

| **Saccharose [mg/ml]** | **Restwassergehalt [%]** | **Glasübergangstemperatur [°C]** |
|---|---|---|
| 20 | 3,09 | 37,30 |
| 40 | 4,19 | 22,51 |
| 60 | 5,37 | 14,44 |

b. Die Tabelle 9 zeigt die Menge an Saccharose in der APE-Lösung (3mg/ml) und den daraus resultierenden Restwassergehalt und die Glasübergangstemperatur der Endprodukte.

**Tabelle 9:**

| **Saccharose [mg/ml]** | **Restwassergehalt [%]** | **Glasübergangstemperatur [°C]** |
|---|---|---|
| 10 | 6,15 | 13,3 |
| 40 | 8,33 | 1,4 |

Die Ergebnisse zeigen, daß beide untersuchten Substanzen, L-Tryptophan und APE, einen trocknungsverbessernden Effekt aufiveisen. Mit L-Tryptophan läßt sich die Glasübergangstemperatur des teilamorphen Produkts bei gleichbleibenden Trockenbedingungen um ca. 45°C steigern. Mit APE ist eine Steigerung der Glasübergangstemperatur bei gleichbleibenden Trockenbedingungen um 20°C möglich.

### Beispiel 6:

### Vakkumtrocknung von anderen Zucker-Aminosäuremischungen

In diesem Versuch wurden binäre Mischungen von Maltosemonohydrat oder Saccharose mit einer L-Aminosäure hergestellt. Hierbei wurden der Zuckerlösung Aminosäuren im Mengenverhältnis Zucker-Aminosäure 5:2 bis 1:1 zugesetzt. Es sollte überprüft werden, ob die jeweilige Aminosäure bei den entsprechenden Zuckern einen trocknungsverbessernden Effekt aufiveist. Die Herstellung, Behandlung und Trocknung der Lösungen erfolgte wie in Beispiel 4 beschrieben. Im einzelnen handelte es sich um folgende Mischungen:
a. Mischungen mit Maltosemonohydrat

**Tabelle 10:**

| **eingesetzte Aminosäure** | **Menge an AS [mg/ml]** | **Zuckermenge [mg/ml]** | **Restwassergehalt [%]** | **Glasübergang [°C]** |
|---|---|---|---|---|
| - | - | 50 | 8,91 | 6,1 |
| L-Arginin | 10 | 50 | 8,10 | 10,3 |
| L-Arginin | 20 | 50 | 8,63 | 7,1 |
| L-Leucin | 20 | 50 | 5,42 | 20,9 |
| L-Leucin | 20 | 20 | 1,87 | 56,05 |
| L-Histidin | 20 | 50 | 9,78 | 5,3 |
| L-Isoleucin | 20 | 20 | 3,12 | 37,9 |
| L-Methionin | 15 | 30 | 7,45 | 9,7 |
| L-Methionin | 20 | 20 | 2,70 | 34,4 |
| L-Valin | 20 | 20 | 4,93 | 18,8 |

b. Mischungen mit Saccharose

**Tabelle 11:**

| **eingesetzte Aminosäure** | **Menge an AS [mg/ml]** | **Zuckermenge [mg/ml]** | **Restwassergehalt [%]** | **Glasübergang [°C]** |
|---|---|---|---|---|
| - | - | 50 | 9,34 | - 1,8 |
| L-Alanin | 15 | 30 | 6,04 | 2,8 |
| L-Alanin | 20 | 20 | 4,46 | 11,7 |
| L-Glycin | 20 | 20 | 4,47 | 5,3 |
| L-Phenylalanin | 20 | 50 | 1,12 | 62,7 |
| L-Serin | 15 | 30 | 11,77 | -15,1 |
| L-Serin | 20 | 20 | 10,61 | - 14,4 |

Die Ergebnisse der Trocknung zeigen, daß L-Histidin keinen positiven Einfluß auf das Trockenverhalten von Zuckern hat (Tab. 10). L-Serin verschlechtert die Trocknung von Zuckern sogar noch weiter (Tab. 11). L-Leucin, L-Isoleucin und L-Methionin haben einen trocknungsverbessernden Effekt (Tab. 10). Dieser wird deutlich, wenn man steigende Mengen dieser Aminosäuren zu der Zuckerlösung hinzu gibt. Bei L-Valin und L-Alanin ist nur bei großen Mengen Aminosäure im Produkt eine Verbesserung der Trocknung festzustellen; L-Arginin und L-Glycin haben einen schwach positiven Einfluß. Die sehr gute Wirkung von L-Phenylalanin auf das Trockenverhalten wird auch bei der binären Mischung mit Saccharose deutlich (Tab. 11). Das Produkt trocknet gut und weist einen sehr hohen Glasübergang auf.

### Vergleichsbeispiel C

### Vakuumtrocknung von Aminosäurelösungen oder von Lösungen von Aminosäuresalzen

Von den einzelnen Aminosäuren oder von Salzen einzelner Aminosäuren wurden Lösungen hergestellt und sterilfiltriert ( 0,22 µm Cellulosenitratfilter). Jeweils 1 ml der Lösung wurde in 2ml-Vials gefüllt und Gefriertrocknungsstopfen aufgesetzt. Die so vorbereiteten Proben wurden bei 20°C unter vermindertem Druck 48 h lang vakuumgetrocknet. Nach der Trocknung wurde der Wassergehalt der Proben nach Karl-Fischer bestimmt und die Glasübergangstemperatur mittels Differentialthermoanalyse (Perkin Elmer DSC7 - Aufheizrate der Proben = 10 K/min) ermittelt. Im einzelnen wurden folgende Lösungen getrocknet und es resultierten die hier angegebenen Restwassergehalte und DSC-Meßergebnisse:
a. Aminosäuren

**Tabelle 12:**

| **Aminosäure** | **Konzentration** | | **Restwassergehalt [%]** | **DSC-Meßergebnis [°C]** |
|---|---|---|---|---|
| | **[mol/l]** | **[mg/ml]** | | |
| L-Alanin | 0,24 | 21,38 | 0,81 | **kristallin** |
| L-Arginin | 0,24 | 41,80 | 0,52 | **kristallin** |
| L-Citrullin | 0,24 | 42,05 | 4,9 | **kristallin** |
| L-Cystein | 0,24 | 29,08 | 2,61 | **kristallin** |
| Glycin | 0,24 | 18,02 | 0,76 | **kristallin** |
| L-Histidin | 0,12 | 18,62 | 0,77 | **kristallin** |
| L-Isoleucin | 0,12 | 15,74 | 1,10 | **kristallin** |
| L-Leucin | 0,12 | 15,74 | 1,62 | **kristallin** |
| L-Lysin | 0,24 | 35,09 | 0,79 | **kristallin** |
| L-Methionin | 0,12 | 17,91 | 1,57 | **kristallin** |
| L-Phenylalanin | 0,12 | 19,82 | 1,53 | **kristallin** |
| L-Prolin | 0,24 | 27,63 | 19,93 | **kristallin** |
| L-Serin | 0,24 | 25,22 | 0,44 | **kristallin** |
| L-Threonin | 0,24 | 28,59 | 0,45 | **kristallin** |
| L-Valin | 0,24 | 28,12 | 0,57 | **kristallin** |

b. Salze von Aminosäuren

**Tabelle 13:**

| **Aminosäure** | **Konzentration** | | **pH-Wert** | **Restwasser- gehalt** | **DSC-meßergebnis** |
|---|---|---|---|---|---|
| | **[mol/l]** | **[mg/ml]** | | **[%]** | **[°C]** |
| L-Arginin | 0,25 | 43,55 | 2,70 | 6,46 | **3,51** |
| HCl | 0,30 | 10,93 | | | |
| L-Arginin | 0,25 | 43,55 | 6,81 | 3,3 | **5,17** |
| H₃PO₄ | 0,15 | 14,70 | | | |
| L-Arginin | 0,25 | 43,55 | 2,89 | 3,24 | 6,67 |
| H₂SO₄ | 0,15 | 14,71 | | | |
| L-Arginin | 0,25 | 43,55 | 2,58 | 2,66 | **kristallin** |
| HNO₃ | 0,30 | 18,90 | | | |
| L-Arginin | 0,25 | 43,55 | 5,24 | 11,04 | **kristallin** |
| Essigsäure | 0,30 | 18,0 | | | |
| L-Asparagin- | 0,12 | 15,97 | 4,97 | 9,65 | **27,7** |
| säure NaOH | 0,12 | 4,8 | | | |
| L-Glutamin- | 0,12 | 17,66 | 5,14 | 14,69 | **5,5** |
| säure NaOH | 0,12 | 4,8 | | | |
| L-Ornithin HCl | 0,24 | 40,47 | 5,39 | 0,4 | **kristallin** |

Das Ergebnis zeigt, daß Aminosäuren nach der Vakuumtrocknung kristallin vorliegen. Nur Salze basischer und saurer Aminosäuren bilden während diesen Trockenbedingungen amorphe Gerüste, die allerdings sehr schlecht trocknen und deren Glasübergangstemperatur unter den gewählten Bedingungen unterhalb der Raumtemperatur liegt.

### Beispiel 7:

### Vakuumtrocknung von L-Arginin-L-Phenylalaninmischungen und einer L-Arginin-L-Isoleucinmischung

In diesem Versuch wurden verschiedene Mischungen von L-Arginin und L-Phenylalanin hergestellt, behandelt, getrocknet und untersucht wie in Vergleichsbeispiel C beschrieben. Im einzelnen wurden folgende binäre Mischungen hergestellt und getrocknet:

**Tabelle 14:**

| **molares Mischungs** | **L-Arginin** | | **L-Phenylalanin** | | **Restwasser- gehalt** | **Glasübergangstemperatur** |
|---|---|---|---|---|---|---|
| **verhältnis** | **[mol/l]** | **[mg/ml]** | **[mol/l]** | **[mg/ml]** | **[%]** | **[°C ]** |
| 1:1 | 0,12 | 20,90 | 0,12 | 19,82 | 2,27 | 59,5 |
| 2:1 | 0,16 | 27,87 | 0,08 | 13,21 | 9,32 | 1,7 |
| 3:1 | 0,18 | 31,35 | 0,06 | 9,91 | 9,40 | 2,8 |
| 4:1 | 0,192 | 33,44 | 0,048 | 7,928 | 9,96 | 1,3 |
| 5:1 | 0,20 | 34,83 | 0,04 | 6,61 | 10,73 | 0,0 |
| 6:1 | 0,206 | 35,88 | 0,034 | 5,61 | 10,03 | 1,3 |
| 7:1 | 0,21 | 36,58 | 0,03 | 4,96 | 11,38 | 1,0 |
| 1:2 | 0,06 | 10,45 | 0,12 | 19,82 | 2,85 | 47,2 |
| 1:3 | 0,04 | 6,97 | 0,12 | 19,82 | 3,43 | 46,2 |
| 1:4 | 0,03 | 5,23 | 0,12 | 19,82 | 3,66 | 43,45 |

Dieser Versuch zeigt, daß es durch Mischen zweier Aminosäuren, die alleine getrocknet kristalline Produkte ergeben, möglich ist, teilamorphe Gerüste herzustellen. Diese trocknen bei ausgewählten Mischungsverhältnissen so gut, daß teilamorphe Gerüste mit einer hohen Glasübergangstemperatur und einem niederen Restwassergehalt resultieren.

Interessant ist, daß es ein optimales Mischungsverhältnis mit dem höchsten Glasübergang gibt. Innerhalb dieses Versuches wurde noch eine Lösung hergestellt, die L-Arginin und L-Isoleucin jeweils 0,15 molar enthielt.

**Tabelle 15:**

| **molares Mischungs** | **L-Arginin** | | **L-Isoleucin** | | **Restwassergehalt** | **Glasübergangstemperatur** |
|---|---|---|---|---|---|---|
| **verhältnis** | **[mol/l]** | **[mg/ml]** | **[mol/l]** | **[mg/ml]** | **[%]** | **[°C]** |
| 1:1 | 0,15 | 26,13 | 0,15 | 19,68 | 1,05 | 53,27 |

Hier resultierte ein Produkt mit einem Glasübergang von 53,27°C und 1,05% Restwassergehalt. Durch Mischen zweier Aminosäuren konnte so ein gut trockenbares teilamorphes Gerüst konstruiert werden; die Glasübergangstemperatur wurde um ca. 50°C gesteigert, verglichen mit Argininsalzen von mineralischen Säuren (Vergleichsbeispiel C).

### Beispiel 8:

### rh-G-CSF in einer L-arginin- und L-phenylalaninhaltigen Maltoserezeptur vakuumgetrocknet

Es wurde eine Lösung mit 50 mg Maltose, 10 mg L-Phenylalanin und 10 mg L-Arginin pro ml hergestellt. Außerdem enthielt diese Lösung 0,1 mg Polysorbat 80 und 0,35 mg rhG-CSF pro ml. Der pH-Wert der Rezeptur wurde mit Salzsäure auf pH 7,4 eingestellt. Die proteinhaltige Lösung wurde unter aseptischen Bedingungen hergestellt und sterilfiltriert (Polyvinylidendifluoridfilter 0,22 µm). Dann wurde jeweils 1 ml der Lösung in 2ml-Vials abgefüllt. Die befüllten und mit Gefriertrocknungsstopfen versehenen Vials wurden dann 48 h bei 20°C isotherm unter vermindertem Druck getrocknet. Es resultierte ein trockenes Produkt mit einem Restwassergehalt von 1,16 % und einer Glasübergangstemperatur von 75°C. Die so hergestellten Proben wurden bei verschiedenen Temperaturen eingelagert und die Proteinstabilität wurde nach verschiedenen Lagerzeiten beurteilt.

Bei rh-G-CSF ist der Anteil an entstandenem Dimer in dem hergestellten Produkt ein gutes Kriterium für die Stabilitätsbeurteilung des Produktes. Daher sind die mittels Ausschlußchromatographie (pro Bedingung 4 Einfachmessungen) ermittelten Mono- und Dimermengen ein Maß für die stabilisierende Wirkung unserer durch Trocknung hergestellten Zubereitungen. Bei der Ausschlußchromatographie werden Proteinmoleküle aufgrund ihrer Teilchengröße in gelöstem Zustand aufgetrennt, d.h. hochmolekulare Bestandteile (Dimere) werden vom rh-G-CSF Monomeren separiert. Die Untersuchung (HP-SEC) wurde an einer HPLC-Anlage von Shimadzu unter Verwendung eines kühlbaren Autosamplers (Waters TM 717) durchgeführt. Als Trennsäule wurde eine TSK-Gel G2000 SW (7,5x300 mm) Säule der Fa. TosoHaas verwendet. Die Detektion der getrennten Bestandteile erfolgte photometrisch bei 214 nm (Photometer Shimadzu LC-GA). Als Fließmittel wurde ein 0.1 m Natrium-Kaliumphosphatpuffer pH 6,2 verwendet, mit dem eine Flußrate von 0,6 ml/min bei Raumtemperatur angelegt wurde. Die zu untersuchenden Proben wurden mit Aqua bidest. so gelöst, daß die Ausgangskonzentration wieder hergestellt war (Zugabe von 1ml). Diese gelösten Proben wurden dann in dem auf 6°C gekühlten Autosampler bis zur Untersuchung aufbewahrt. Die Einspritzmenge einer Probe betrug 20µl (= 7 µg G-CSF), die Laufzeit einer Probe 32 min. Die Auswertung der Ergebnisse erfolgte unter Benutzung eines G-CSF-Arbeitsstandards. Um die Produkte zusätzlich qualitativ beurteilen zu können, wurde außerdem bei jeder Gehaltsbestimmung eine SDS-Gelelektrophorese mit silver stain Färbung durchgeführt. Die Ergebnisse zur SDS-Gelelektrophorese sind in Beispiel 8 b Abb.9 dargestellt. In wässrigen Lösungen denaturiert das Protein bei Temperaturen zwischen 45° und 47°C innerhalb weniger Stunden vollständig. Mit dieser Rezeptur gelang es, das Protein selbst bei 50°C Lagertemperatur über Wochen zu stabilisieren.

### a. Stabilität von rhG-CSF in einer vakuumgetrockneten Maltoserezeptur mit L-Arginin und L-Phenylalanin.

**Tabelle 16:**

| Angegeben sind die Monomergehalte in % wie in der HP-SEC erhalten | | | | | |
|---|---|---|---|---|---|
| **Lagerzeit [Wochen]** | **Lagertemperatur** | | | | |
| | **KS** | **RT** | **30°C** | **40°C** | **50°C** |
| 0 | - | 99,83 % | - | - | - |
| 5 | 99,94 % | 99,93 % | 99,86 % | 99,89 % | 99,87 % |
| 13 | 99,83 % | 99,86 % | 99,88 % | 99,83 % | 99,83 % |
| 26 | 99,76 % | 99,75 % | 99,55 % | 99,36 % | 99,21 % |
| 39 | 99,68 % | 96,73 % | 96,40 % | 93,81 % | 89,27 % |
| 52 | 98,34% | 94,81 % | 94,70 % | 91,27 % | 86,27 % |
| KS = Kühlschranktemperatur = 4 - 6°C RT = Raumtemperatur = 20 - 22°C | | | | | |

Die Ergebnisse der Ausschlußchromatographie zeigen deutlich, daß es mit dieser Rezeptur möglich ist, das Protein rhG-CSF über einen längeren Zeitraum in einer solchen vakuumgetrockneten Zubereitung zu stabilisieren. Der Versuch zeigt, daß es möglich ist, rhG-CSF in einer vakuumgetrockneten, teilamorphen, L-arginin- und L-phenylalaninhaltigen Maltoserezeptur unterhalb der Glasübergangstemperatur über längere Zeit zu stabilisieren.

### b. Übersicht der Ergebnisse der SDS-Gelelektrophorese aller Rezepturen, die den Wirkstoff rh-G-CSF enthielten.

Zunächst wurden SDS-haltige Polyacrylamidgele, deren Trenngel 15% Acrylamid und deren Sammelgel 3% Acrylamid und 1% Natrium-Dodecylsulfat (SDS) enthielt, hergestellt. Die Vorbereitung der Proben erfolgte so, daß aus 3 Injektionsflaschen 1 Mischmuster hergestellt wurde. Anschließend wurde diese Probelösung mit einem dithiothreithaltigen (DTT) und bromphenolblauhaltigem Probenpuffer verdünnt, so daß eine rh-G-CSF-Konzentration von 150 µg/ml entstand. Die Proben wurden 5 min bei 95°C im vorgeheizten Heizblock denaturiert. Als Eichproteine wurden die Proteine "Combithek Eichproteine für die Chromatographie MG 18000 - 300000" von Boehringer Mannheim verwendet. Diese wurden genauso wie die rh-G-CSF-Proben vorbereitet und behandelt. Außerdem wurde ein rh-G-CSF Arbeitsstandard präpariert, der als Vergleich eingesetzt wurde. Die Gelelektrophorese wurde mittels einer Midget Gelelektrophoresis Unit (Pharmacia - LKB 2050) und einem dazugehörigen Spannungsgerät durchgeführt. Nachdem der Elektrophoresepuffer eingefüllt worden war, wurde in jede Geltasche 20 µl Probe (d.h. 3 µg rh-G-CSF) eingefüllt. Nach Verschließen der Elektrophoresekammer und Anstellen der Wasserkühlung wurde eine Spannung von 80 V angelegt, die nachdem das Sammelgel durchlaufen war, auf 130 V erhöht wurde. Kurz bevor die Bromphenolblaubande das Ende des Gels erreichte, wurde die Elektrophorese beendet. Die Gele wurden aus der Kammer entfernt und mit Aqua bidest. kurz gewaschen. Dann wurde eine silver-stain-Färbung mit einem Daiichi 2D-silver-stain II Kit gemäß der dort enthaltenen Anweisung durchgeführt. Nach Ende der Färbung erfolgte die optische Begutachtung der Gele.

**Tabelle 17:**

| Ergebnis der Gelelektrophorese | | |
|---|---|---|
| **Spur** | **Zubereitung** | **Sichtergebnis** |
| 1 | Eichproteine | |
| 2 | **Beispiel. 8:** L-arginin- und L-phenylalaninhaltige Maltoserezeptur | nur Monomere |
| 3 | **Vergleichsbeispiel D:** Trocknung ohne Hilfsstoffe | Monomere und Dimere |
| 4 | **Vergleichsbeispiel E:** reine Maltoserezeptur | Monomere, Dimere und Trimere |
| 5 | Eichproteine | |
| 6 | **Beispiel 9:** L-arginin- und L-phenylalaninhaltige, zuckerfreie Rezeptur | nur Monomere |
| 7 | **Vergleichsbeispiel F:** L-Argininrezeptur mit Phosphorsäure | Monomere und Dimere |
| 8 | **Vergleichsbeispiel G:** kristalline L-Valin - L-Glycinrezeptur | Monomere, Dimere und Abbauprodukte |

Die Rezepturen der Beispiele 8 und 9 zeigen bei dieser Untersuchung nur Monomere. Bei Vergleichsbeispiel D und F sind neben dem Monomer noch Dimere, bei Beispiel E außerdem noch zusätzlich Trimere zu erkennen. In der kristallinen L-Valin - L-Glycin Zubereitung Vergleichsbeispiel G sieht man außerdem noch 2 Abbauprodukte, deren Molekülmasse kleiner ist, als die des Monomers und 2 schwache Banden von Abbauprodukten, deren Molekülmasse zwischen der des Monomers und des Dimers liegt.

Anhand dieser empfindlichen Methode konnten sehr gut Abbau- und Aggregationsprodukte des Monomers, die in Mengen > 1% vorlagen, sichtbar gemacht werden.

### Vergleichsbeispiel D

### Vakuumtrocknung von rhG-CSF ohne Zugabe von Hilssloffen

Bei diesem Versuch wurde eine Lösung hergestellt, die nur das Protein rhG-CSF in einer Konzentration von 0,35 mg/ml in einem verdünnten Phosphatpuffer ( ca. 0,01m) enthielt. Diese Proteinlösung wurde hergestellt, behandelt und analysiert wie in Beispiel 8 beschrieben. Bei dieser Zubereitung war es aus technische Gründen nicht möglich, bei den Endprodukten den Restwassergehalt und die Glasübergangstemperatur zu bestimmen. Stabilitätsdaten von rhG-CSF ohne Hilsstoffe vakuumgetrocknet

**Tabelle 18:**

| Angegeben sind die Monomeranteile in % wie in der HP-SEC erhalten | | | |
|---|---|---|---|
| **Lagerzeit [Wochen]** | **Lagertemperatur** | | |
| | **KS** | **RT** | 40°C |
| 0 | - | 97,84 % | - |
| 5 | 94,90 % | 94,53 % | 93,96 % |
| 13 | 91,31 % | 89,66 % | 78,23 % |
| 26 | 80,06 % | 73,60 % | 52,22 % |
| KS = Kühlschranktemperatur = 4- 6°C RT = Raumtemperatur = 20 - 22°C | | | |

Die Stabilitätsdaten des reinen Proteins zeigen sehr deutlich die stabilisierende Wirkung der Hilfsstoffe der in Beispiel 8 beschriebenen Rezeptur. Auch bei dieser Rezeptur wurde bei jeder Untersuchung eine SDS-Gelelektrophorese mit silverstain Färbung durchgeführt. Ergebnisse siehe Beispiel 8 b.

### Vergleichsbeispiel E

### rhG-CSF in einer reinen Maltoserezeptur vakuumgetrocknet

Es wurde eine Lösung mit 50mg Maltosemonohydrat, 0,1mg Polysorbat 80 und 0,35 mg rhG-CSF pro ml hergestellt. Der pH-Wert der Rezeptur wurde mit Natronlauge auf 7,4 eingestellt. Die Ausgangslösung und die Endprodukte wurden hergestellt, behandelt und analysiert wie in Beispiel 8 beschrieben. Wie schon Beispiel 1 zeigt, ist es schwierig, Maltose ohne Zusatz von Aminosäuren innerhalb von 48 h auf geringe Restfeuchten zu trocknen. Deshalb resultieren Produkte mit einem Restwassergehalt von 10,43 % und einer Glasübergangstemperatur von - 2C. Bei den Lagertemperaturen, also oberhalb des Glasübergangs, lag kein amorphes, sprödes Glas sondern eine hochviskose, zähelastische Masse vor. Stabilität von rhG-CSF in einer vakuumgetrockneten Maltosezubereitung ohne Aminosäuren.

**Tabelle 19:**

| Angegeben sind die Monomergehalte in % wie in der HP-SEC erhalten. | | | | | |
|---|---|---|---|---|---|
| **Lagerzeit [Wochen]** | **Lagertemperatur** | | | | |
| | **KS** | **RT** | **30 C** | **40 C** | **50 C** |
| 0 | - | 97,99 % | - | - | - |
| 5 | 98,26 % | 96,82 % | 93,91 % | 67,45 % | 42,63 % |
| 13 | 97,15 % | 90,49 % | 73,70 % | 30,05 % | 18,52 % |
| 26 | 97,05 % | 88,23 % | 71,32 % | 22,30 % | 15,27 % |

Zu den Ergebnissen der SDS-Gelelektrophorese siehe Beispiel 8 b. Das Ergebnis zeigt, daß es nicht vorteilhaft ist, rhG-CSF in einer Zuckermasse ohne Aminosäuren aufzubewahren. Die Stabilität ist deutlich geringer, als bei vakuumgetrocknetem Bulk (Vergleichsbeispiel D) und einer optimierten, vakuumgetrockneten Rezeptur (Beispiel 8).

Dieser Versuch zeigt deutlich die Notwendigkeit der Zugabe von Aminosäuren zu Zuckern bei der Vakuumtrocknung, um Produkte mit hohen Glasübergängen zu erhalten, in denen das Protein dann durch das amorphe Hilfsstoffgerüst stabilisiert wird. Eine Lagerung der Produkte unterhalb der Glasübergangstemperatur erweist sich als Notwendigkeit fiir die Stabilisierung des Wirkstoffs. Zu bemerken ist noch, daß in den Proben, die bei 40 und 50 C eingelagert waren, bereits nach 4 Wochen die Maltose vollständig auskristallisiert war. Solche physikalischen Veränderungen in den Proben während der Lagerung sind zu vermeiden; sie beschleunigen die Abnahme des Monomergehaltes.

### Beispiel 9:

### rh-G-CSF in einer zuckerfreien L-Arginin-L-Phenylalaninrezeptur vakuumgetrocknet

Eine Lösung mit 20 mg L-Arginin und 20 mg L-Phenylalanin, 0,1 mg Polysorbat 80 und 0,35 mg rh-G-CSF pro ml wurde hergestellt. Nachdem der pH-Wert mit Salzsäure auf pH 7,4 eingestellt war, wurde die Lösung behandelt, getrocknet und analysiert wie in Beispiel 8 beschrieben. Nach Ende der Trocknung lag ein homogenes Produkt mit einer Glasübergangstemperatur von 77,0°C und einem Restwassergehalt von 1,30% vor. Stabilität von rh-G-CSF in einer vakuumgetrockneten Arginin-Phenylalaninrezeptur.

**Tabelle 20:**

| Angegeben sind die Monomeranteile wie in der HP-SEC erhalten | | | | | | |
|---|---|---|---|---|---|---|
| **Lagerzeit** | **Lagertemperaturen** | | | | | |
| **[Wochen]** | **KS** | **RT** | **30°C** | **40°C** | **60°C** | **80°C** |
| 0 | - | 99,57% - | | - | - | - |
| 4 | 99,36 % | 99,36 % | 99,50 % | 99,81 % | 96,56 % | 1,44% |
| 13 | 99,17% | 99,31% | 99,40% | 99,64% | - | - |
| 26 | 98,64 % | 98,62 % | 96,52 % | 91,06 % | - | - |
| 39 | 99,64 % | 94,18 % | 88,99 % | 79,05 % | - | - |
| KS = Kühlschranlemperatur= 4-6°C RT = Raumtemperatur = 20-22°C | | | | | | |

Die Ergebnisse der Haltbarkeitsuntersuchung zeigen deutlich, daß es mit dieser Rezeptur möglich ist, das Protein rh-G-CSF über einen längeren Zeitraum in einer teilamorphen, vakuumgetrockneten Aminosäurenzubereitung zu stabilisieren, solange die Lagertemperaturen deutlich unterhalb der Glasübergangstemperaturen liegen (vergleiche auch Vergleichsbeispiel C). Die Lagerung bei 80°C versus der Lagerung bei 60°C zeigt dieses Phänomen bezüglich des Glasübergangs bei 77°C.

Um die Produkte zusätzlich qualitativ beurteilen zu können, wurde außerdem bei jeder Gehaltsbestimmung eine SDS-Gelelektrophorese mit silver stain Färbung durchgeführt. Die Ergebnisse dieser SDS-Gelelektrophorese sind in Beispiel 8 b dargestellt. Die selbe Rezeptur wurde auch bei verschiedenen Temperaturen 1 Jahr lang gelagert. Das Ergebnis ist in Tabelle 20a gezeigt.

| | Wassergehalt [%] | Tg [°C] | Monomergehalt G-CSF [%] |
|---|---|---|---|
| Start | 0,77 | 82,1 | 99,82 |
| nach 52 Wochen: | | | |
| KS | 1,20 | 79,52 | 98,34 |
| RT | 2,08 | 69,47 | 94,81 |
| 30° | 2,21 | 67,91 | 94,70 |
| 40° | 2,32 | 67,36 | 91,27 |
| 50° | 2,40 | 68,63 | 86,26 |
| KS = Kühlschranktemperatur = 4-6°C RT = Raumtemperatur = 20-22°C | | | |

Der Versuch zeigt, daß es möglich rh-CSF in einer vakuumgetrockneten, teilamorphen L-Arginin-L-Phenylalaninrezeptor unterhalb de Glasübergangstemperatur zu stabilisieren.

### Vergleichsbeispiel F

### rh-G-CSF in einer vakuumgetrockneten L-Argininrezeptur mit Phosphorsäure

Es wurde eine Lösung mit 40 mg L-Arginin, 0,1 mg Polysorbat 80 und 0,35 mg rh-G-CSF pro ml hergestellt. Nachdem der pH-Wert mit Phosphorsäure auf pH 7,4 eingestellt war, wurde die Lösung behandelt, getrocknet und analysiert wie in Beispiel 8 beschrieben. Das getrocknete Endprodukt wies einen Restwassergehalt von 3,59 % und eine Glasübergangstemperatur von 8,6°C auf. Dieses Produkt lag folglich nach Abschluß der Trocknung bei Raumtemperatur nicht als sprödes, amophes Glas, sondern als hochviskose, zähplastische Masse vor. Stabilität von rh-G-CSF in einer vakuumgetrockneten L-Argininrezeptur.

**Tabelle 21:**

| Angegeben sind die Monomeranteile in % wie in der HP-SEC erhalten | | | | | | | |
|---|---|---|---|---|---|---|---|
| **Lagerzeit [Wochen]** | **Lagertemperaturen** | | | | | | |
| | **-20°C** | **KS** | **RT** | **30°C** | **40°C** | **60°C** | **80°C** |
| 0 | - | - | 99,60% | - | - | - | - |
| 4 | 99,57% | 99,60% | 99,37% | 99,34% | 99,20% | 89,46% | 31,81% |
| 13 | 99,75 % | 98,17% | 98,06 % | 97,31 % | 93,41 % | - | - |
| 33 | 99,28 % | 99,30 % | 99,21 % | 97,86 % | 93,02 % | - | - |

Es wird nicht der stabilisierende Effekt erreicht, den man in einem trockenen, teilamorphen Glas erhält (Beispiel 8 und 17). Dies zeigt die Bedeutung der geschickten Mischung von Hilfsstoffen, um deren Trockenverhalten während der Vakuumtrocknung zu verbessern und so bei Raumtemperatur teilamorphe Gläser vor sich zu haben. Vor allem bei höheren Temperaturen (30° und 40°C) wird dies deutlich. Die Stabilität ist gegenüber dem vakuumgetrockneten Wirkstoff ohne Hilfsstoffe in dieser Masse erhöht (Vergleiche Vergleichsbeispiel D). Um die Produkte zusätzlich qualitativ beurteilen zu können, wurde außerdem bei jeder Gehaltsbestimmung eine SDS-Gelelektrophorese mit silver stain Färbung durchgeführt. Die Ergebnisse dieser SDS-Gelelektrophorese sind in Beispiel 8 b dargestellt.

### Vergleichsbeispiel G

### rh-G-CSF in einer kristallinen L-Valin - Glycinrezeptur vakuumgetrocknet

0,35 mg rh-G-CSF pro ml wurden in eine Lösung gegeben, die je 20 mg L-Valin und Glycin und 0,1 mg Polysorbat 80 pro ml enthielt und deren pH-Wert mit Natronlauge auf pH 7,4 eingestellt war. Die fertig angesetzte Lösung wurde behandelt, getrocknet und analysiert wie in Beispiel 8 beschrieben. Die Untersuchung nach Ende der Trocknung zeigte, daß es sich bei den fertigen Mustern um ein kristallines Produkt mit einem Restwassergehalt von 0,82 % handelte. Stabilität von rh-G-CSF in einer vakuumgetrockneten, kristallinen L-Valin -Glycinrezeptur.

**Tabelle 22:**

| Angegeben sind die Monomeranteile in % wie in der HP-SEC erhalten | | | | | | |
|---|---|---|---|---|---|---|
| **Lagerzeit [Wochen]** | **Lagertemperaturen** | | | | | |
| | **KS** | **RT** | **30°C** | **40°C** | **60°C** | **80°C** |
| 0 | - | 94,36 % | - | - | - | - |
| 4 | 89,93 % | 89,66 % | 84,26 % | 72,47 % | 44,54 % | 27,44 % |
| 13 | 74,14% | 73,91 % | 64,90% | 46,82% | - | - |
| 33 | 73,75 % | 70,04 % | 54,93 % | 40,40 % | - | - |
| KS = Kühlschranktemperatur= 4-6°C RT = Raumtemperatur = 20-22°C | | | | | | |

Dieses Ergebnis zeigt deutlich, daß eine kristalline Aminosäurerezeptur, selbst bei niedrigen Restwassergehalten, nicht in der Lage ist, rh-G-CSF zu stabilisieren. Verglichen mit dem ohne Hilfsstoffe vakuumgetrockneten rh-G-CSF zeigt sich deutlich der destabilisierende Effekt einer solchen Zubereitung ( siehe Vergleichsbeispiel D).

Um die Produkte zusätzlich qualitativ beurteilen zu können, wurde außerdem bei jeder Cehaltsbestimmung eine SDS-Gelelektrophorese mit silver stain Färbung durchgeführt. Ergebnisse dieser SDS-Gelelektrophorese sind in Beispiel 8 b dargestellt.

### Beispiel 10:

### Vakuumtrocknung von Erythropoietin in einer L-arginin- und L-phenylalaninhaltigen Saccharoserezeptur

Eine Lösung mit 50 mg Saccharose, je 10 mg L-Arginin und L-Phenylalanin und 0,1 mg Polysorbat 20 pro ml wurde hergestellt. In diese Lösung wurden 5000 U Erythropoietin (EPO) pro ml zugegeben und der pH-Wert mit Phosphorsäure auf pH 7,2 eingestellt. Die Lösung wurde behandelt und getrocknet wie in Beispiel 8 beschrieben. Es resultierte ein trockenes, teilamorphes Produkt mit einem Restwassergehalt von 0,56 % und einer Glasübergangstemperatur von 86,6°C. Bei EPO ist der Anteil an entstandenem Dimer in dem hergestellten Produkt ein gutes Kriterium für die Stabilitätsbeurteilung des Produktes. Daher sind die mittels Ausschlußchromatographie (pro Bedingung 3 Einfachmessungen) ermittelten Mono- und Dimermengen ein Maß für die stabilisierende Wirkung unserer durch Trocknung hergestellten Zubereitungen.

Bei der Ausschlußchromatographie werden Proteinmoleküle aufgrund ihrer Teilchengröße in gelöstem Zustand aufgetrennt, d.h. hochmolekulare Bestandteile (Dimere) werden vom EPO Monomeren separiert. Die Untersuchung (HP-SEC) wurde an einer HPLC-Anlage von Shimadzu unter Verwendung eines Autosamplers (Gilson Abimed 231) durchgeführt. Als Trennsäule wurde eine TSK-Gel G3000 SWXL (7,8x300 mm) Säule der Fa. TosoHaas verwendet. Die Detektion der getrennten Bestandteile erfolgte photometrisch bei 280 nm (Merck Fluorescence Spectrophotometer 820 FP). Als Fließmittel wurde ein 0,41m Natrium-Kaliumphosphatpuffer mit Natriumchlorid pH 7,3 verwendet, mit dem eine Flußrate von 0,6 ml/min bei Raumtemperatur angelegt wurde. Die zu untersuchenden Proben wurden mit Aqua bidest. so gelöst, daß die Ausgangskonzentration wieder hergestellt war (Zugabe von 1ml). Diese gelösten Proben wurden dann im Autosampler bis zur Untersuchung aufbewahrt. Die Einspritzmenge einer Probe betrug 100µl (= 2 µg EPO), die Laufzeit einer Probe 25 min. Die Auswertung der Ergebnisse erfolgte unter Benutzung eines EPO-Arbeitsstandards.

Stabilität von EPO in einer vakuumgetrockneten, L-arginin- und L-phenylalaninhaltigen Saccharoserezeptur.

**Tabelle 23:**

| Angegeben sind die Monomergehalte in % wie in der HP-SEC erhalten | | | |
|---|---|---|---|
| **Lagerzeit [Wochen]** | **Lagertemperatur** | | |
| | **KS** | **RT** | **40°C** |
| 0 | - | 100% | - |
| 4 | 100% | 100% | 100% |
| 9 | 100% | 100% | 100% |
| 13 | 100 % | 100 % | 100 % |
| 26 | 100 % | 100 % | 99,9 % |
| KS = Kühlschranktemperatur= 4-6°C RT = Raumtemperatur = 20-22°C | | | |

Das Ergebnis zeigt, daß es möglich ist, EPO mittels Vakuumtrocknung mit der hier gewählten Hilfsstoffkombination zu stabilisieren. Um die Produkte zusätzlich qualitativ beurteilen zu können, wurde außerdem bei jeder Gehaltsbestimmung eine SDS-Gelelektrophorese mit silver stain Färbung durchgeführt. Die Herstellung der Gele, die Durchfiihrung der Elektrophorese und die Färbung der Gele erfolgten wie in Beispiel 8 b beschrieben. Die Vorbereitung der Proben erfolgte so, daß aus 3 Injektionsflaschen 1 Mischmuster hergestellt wurde. Anschließend wurde diese Probelösung mit einem bromphenolblauhaltigem Probenpuffer verdünnt, so daß eine EPO-Konzentration von 20 µg/ml entstand. Die Proben wurden 5 min bei 95°C im vorgeheizten Heizblock denaturiert. Als Eichproteine wurden ein "Bio-Rad Standard Low"verwendet. Dieser wurde genauso wie die EPO-Proben vorbereitet und behandelt. Außerdem wurde ein EPO-Arbeitsstandard präpariert, der als Vergleich eingesetzt wurde. Die Gelelektrophorese wurde mittels einer Midget Gelelektrophoresis Unit (Phannacia - LKB 2050) und einem dazugehörigen Spannungsgerät durchgeführt. Nachdem der Elektrophoresepuffer eingefüllt worden war, wurde in jede Geltasche 20 µl Probe (d.h. 400 ng EPO) eingefüllt. Nach Ende der Färbung erfolgte die optische Begutachtung der Gele und die Gele wurden fotografiert. Anhand dieser empfindlichen Methode konnten sehr gut Abbau- und Aggregationsprodukte des Monomers, die in Mengen > 1% vorlagen, sichtbar gemacht werden.

### Ergebnis der Elektrophorese

Bei der hier beschriebenen Rezeptur war bei allen Proben nach 9 Wochen im Gel nur eine Monomerbande entsprechend dem Arbeitsstandard zu erkennen. Dies unterstreicht die Stabilität des Proteins in dieser Zubereitung.

### Vergleichsbeispiel H

### Vakuumtrocknung von Erythropoietin ohne Zugabe von Hilfsstoffen

In diesem Versuch wurde eine Ausgangslösung hergestellt, die nur den Wirkstoff EPO (50000 U/ml) in einem verdünnten Phosphatpuffer (ca. 5 mM) enthielt. Die Lösung wurde hergestellt, behandelt und getrocknet wie in Beispiel 8 beschrieben.

Bei dieser Zubereitung war es aus technische Gründen nicht möglich, bei den Endprodukten den Restwassergehalt und die Glasübergangstemperatur zu bestimmen, da die vorhandenen Mengen in einem Vial zu gering waren (ca. 0,2 mg). Die Stabilität des Proteins wurde, wie in Beispiel 10 beschrieben, mittels Ausschlußchromatographie beurteilt.

Stabilität von EPO ohne Hilfsstoffe vakuumgetrocknet.

**Tabelle 24:**

| Angegeben sind die Monomergehalte in % wie in der HP-SEC erhalten | | | |
|---|---|---|---|
| **Lagerzeit [Wochen]** | **Lagertemperatur** | | |
| | **KS** | **RT** | **40° C** |
| 0 | - | 99,5% | - |
| 4 | 98,6% | 94,4% | 88,0% |
| 9 | 96,0% | 89,0% | 75,0% |
| 13 | 95,7 % | 87,0 % | 76,3 % |
| 26 | 94,7 % | 88,2 % | 66,6 % |
| KS = Kühlschranktemperatur= 4-6° C RT = Raumtemperatur = 20-22° C | | | |

Ebenfalls wurde bei jeder Gehaltsbestimmung eine SDS-Gelelektrophorese mit silver stain Färbung durchgeführt. Die Herstellung der Gele, Vorbereitung der Proben, die Durchführung der Elektrophorese und die Färbung der Gele erfolgten wie in Beispiel 10 beschrieben. Nach dem Färben der Gele konnte man bei den Proben jeder Lagertemperatur neben der Monomerbande, entsprechend der Bande des Arbeitsstandards, deutlich eine Dimerbande erkennen. Dieser Versuch macht die stabilisierende Wirkung der in Beispiel 10 eingesetzten Hilfsstoffkombination deutlich. Die Stabilität des reinen Wirkstoffes in diesem Beispiel ist gegenüber der Stabilität des Wirkstoffes in der Rezeptur Beispiel 10 deutlich erniedrigt; es ist eine Dimerbildung zu beobachten. Je höher die Lagertemperatur ist, um so deutlicher wird der protektive Effekt der gewählten Kombination an Hilfsstoffen in Versuch 10.

### Beispiel 11:

### Lactatdehydrogenase in einer Maltose-L-Arginin-L-Phenylalaninrezeptur vakuum-getrocknet

Es wurde eine Lösung mit 50 mg Maltosemonohydrat, 10 mg L-Arginin und 10 mg L-Phenylalanin pro ml hergestellt. Dieser Lösung wurde Lactatdehydrogenase (LDH) zugefügt, so daß eine Proteinaktivität von 165 U/ml resultierte. Der pH-Wert der Lösung wurde mit Phosphorsäure auf pH 7,0 eingestellt. Die Lösung wurde hergestellt, behandelt und getrocknet wie in Beispiel 8 beschrieben. Nach der Trocknung lag ein homogenes Produkt mit einer Glasübergangstemperatur von 96°C und einem Restwassergehalt von 0,82% vor. Die fertigen Proben wurden bei verschiedenen Temperaturen eingelagert und die Proteinaktivität nach verschiedenen Lagerzeiten beurteilt. Bei der LDH wurde die enzymatische Aktivität als Maß für die Proteinstabilität bestimmt. Diese Bestimmung erfolgt photometrisch. In einer Probelösung wird Pyruvat mit NADH unter katalytischer Wirkung der LDH zu Lactat und NAD reduziert. Die Abnahme des NADH-Gehaltes in der Lösung kann photometrisch verfolgt werden ( λ= 365 nm; ε = 3,4 cm²/µmol ). Die Aktivität wurde in 100 oder 200fach verdünnten Ausgangslösungen in einer Kunststoffküvette (Schichtdicke = 1cm) gemessen (Perkin-Elmer 552 UV/VIS Spectrophotometer). Aus der Abnahme pro Zeiteinheit kann dann die Proteinakivität der LDH berechnet werden. Stabilität von LDH in einer vakuumgetrockneten, arginin- und phenylalaninhaltigen Maltoserezeptur. Die Aktivität der Ausgangslösung entsprach einem Wert von 100%.

**Tabelle 25:**

| Angegeben ist die Aktivität in % wie in der Untersuchung erhalten | | | | | |
|---|---|---|---|---|---|
| **Lagerzeit [Wochen]** | **Lagertemperatur** | | | | |
| | **KS** | **RT** | **30°** | **40°** | **60°** |
| 0 | - | 85,3 % | - | - | - |
| 5 | 87,81 % | 87,45 % | 81,15 % | 80,42 % | 64,42 % |
| 13 | 83,28% | 79,41 % | 78,79% | 61,74% | - |
| KS = Kühlschranktemperatur = 4-6°C RT = Raumtemperatur = 20-22°C | | | | | |

Der Versuch zeigt deutlich die stabilisierende Wirkung der Rezeptur fiir das sehr empfindliche Protein LDH.

### Vergleichsbeispiel I

### Vakuumtrocknung von Lactatdehydrogenase ohne Zugabe von Hilfsstoffen

In diesem Versuch wurde eine Lösung des reinen Wirkstoffes Lactatdehydrogenase (LDH) mit einer Aktivität von 136 U/ml in einem verdünnten Phosphatpuffer ( 8 mM) hergestellt. Die Lösung wurde hergestellt, behandelt und getrocknet wie in Beispiel 8 beschrieben. Bei dieser Zubereitung war es aus technischen Gründen nicht möglich, bei den Endprodukten den Restwassergehalt und die Glasübergangstemperatur zu bestimmen, da die vorhandenen Mengen in einem Vial zu gering waren (ca. 0,2 mg). Die Stabilität des Proteins wurde, wie in Beispiel 11 beschrieben, beurteilt. Stabilität von LDH ohne Hilfsstoffe vakuumgetrocknet. Die Aktivität der Ausgangslösung entsprach einem Wert von 100%.

**Tabelle 26:**

| Angegeben ist die Aktivität in % wie in der Untersuchung erhalten | | | |
|---|---|---|---|
| **Lagerzeit [Wochen]** | **Lagertemperatur** | | |
| | **KS** | **RT** | **40° C** |
| 0 | - | 64,52 % | - |
| 5 | 66,54 % | 23,03 % | 1,57 % |
| 13 | 50,63 % | 6,81 % | 0% |
| KS = Kühlschranktemperatur= 4-6° C,RT = Raumtemperatur = 20-22° C | | | |

Dieser Versuch macht die stabilisierende Wirkung der in Beispiel 11 eingesetzten Hilfsstoffkombination deutlich. Die Stabilität des reinen Wirkstoffes in diesem Beispiel ist gegenüber der Stabilität des Wirkstoffes in der Rezeptur Beispiel 11 deutlich erniedrigt. Je höher die Lagertemperatur ist, um so deutlicher wird der protektive Effekt der gewählten Kombination an Hilfsstoffen in Versuch 11. Der Unterschied zwischen der Stabilität von reinem Protein und dem Protein in einer Hilfsstoffkombination getrocknet ist bei der LDH am deutlichsten.

### Beispiel 12:

### Lactatdehydrogenase in einer zuckerfreien, vakuumgetrockneten L-Arginin - L-Phenylalanin-rezeptur

Eine Stammlösung mit 20 mg L-Arginin und 20 mg L-Phenylalanin pro ml wurde hergestellt. Dieser wurde nach Einstellung des pH-Wertes auf pH 7,0 mit Phosphorsäure Lactatdehydrogenase (LDH) zugefügt, so daß eine Ausgangslösung mit einer Proteinaktivität von 168 U/ml resultierte. Die Lösung wurde hergestellt, behandelt und getrocknet wie in Beispiel 8 beschrieben. Nach der Trocknung lag ein homogenes Produkt mit einer Glasübergangstemperatur von 103,9°C und einem Restwassergehalt von 1,18% vor. Die fertigen Proben wurden bei verschiedenen Temperaturen eingelagert und die Proteinaktivität nach verschiedenen Lagerzeiten beurteilt. Die Proteinanalytik wurde durchgeführt wie in Beispiel 11 beschrieben. Stabilität von LDH in einer vakuumgetrockneten, zuckerfreien L-Arginin - L-Phenylalaninzubereitung. Die Aktivität der Ausgangslösung vor der Trocknung entsprach einem Wert von 100%.

**Tabelle 27:**

| Angegeben ist die Enzymaktivität in % wie im Aktivitätstest erhalten | | | | | |
|---|---|---|---|---|---|
| **Lagerzeit [Wochen]** | **Lagertemperaturen** | | | | |
| | **KS** | **RT** | **30°C** | **40°C** | **60°C** |
| 0 | - | 80,36 % | - | - | - |
| 4 | 79,70 % | 82,08 % | 79,34 % | 77,62 % | 70,54 % |
| 13 | 82,25% | 76,11 % | 75,21 % | 73,06 % | - |

Der Versuch zeigt deutlich die stabilisierende Wirkung dieser Aminosäurezubereitung über den ganzen untersuchten Temperaturbereich. Die Stabilität der LDH ist gegenüber der Trocknung des reinen Wirkstoffes (Vergleichsbeispiel I) deutlich erhöht.

### Vergleichsbeispiel J:

### Lactatdehydrogenase in einer kristallinen L-Valin - Glycinrezeptur vakuumgetrocknet

Eine Stammlösung mit 20 mg L-Valin und 20 mg Glycin pro ml wurde hergestellt. Dieser wurde nach Einstellen des pH-Wertes auf pH 7,0 mit NaOH-Lösung Lactat-dehydrogenase (LDH) zugefügt, so daß eine Ausgangslösung mit einer Proteinaktivität von 147 U/ml resultierte. Die Lösung wurde hergestellt, behandelt und getrocknet wie in Beispiel 8 beschrieben. Nach der Trocknung lag ein homogenes, vollkommen kristallines Produkt mit einem Restwassergehalt von 1,12 % vor. Die fertigen Proben wurden bei verschiedenen Temperaturen eingelagert und die Proteinaktivität wurde nach bestimmten Lagerzeiten, wie in Beispiel 11 beschrieben, bestimmt. Stabilität von LDH in einer vakuumgetrockneten, vollkommen kristallinen L-Valin - Glycinrezeptur. Die Aktivität der Ausgangslösung vor der Trocknung entsprach einem Wert von 100%.

**Tabelle 28:**

| Angegeben ist die Enzymaktivität in % wie im Aktivitätstest erhalten | | | | | |
|---|---|---|---|---|---|
| **Lagerzeit [Wochen]** | **Lagertemperaturen** | | | | |
| | **KS** | **RT** | **30°C** | **40°C** | **60°C** |
| 0 | | 9,26 % | | | |
| 5 | 3,69 % | 2,11 % | 1,09% | 0,80% | 0,0% |
| 13 | 0,08% | 0,01% | 0% | 0% | 0% |

Dieser Versuch zeigt deutlich, daß eine kristalline Aminosäurenrezeptur während der Vakuumtrocknung einen sehr negativen Einfluß auf das Enzym hat. Schon während der Trocknung, dies bedeutet während der Bildung des kristallinen Gerüstes, gehen schon 90% der Aktivität verloren. Auch bei der Einlagerung bei verschiedenen Temperaturen kann die noch vorhandene Aktivität nicht erhalten werden. Nach 5 Wochern haben sich die Startwerte der Proben noch weiter verschlechtert. Eine vollkommen kristalline Aminosäurenrezeptur ist zur Stabilisierung von LDH also gänzlich ungeeignet.

### Beispiel 13

In diesem Versuch wurden eine reine Maltoselösung (50 mg/ml) und eine Lösung mit Maltose und Phenylalanin (40mg/ml Maltose und 10 mg/ml Phenylalanin) vakuumgetrocknet. Parallel dazu wurden derartige Zubereitungen mit Zusatz von 10 µg/ml rh-ngf oder 100 µg/ml PTH(1-37) oder 500 µg/ml Ularitide hergestellt. Die Lösungen wurden nach der Herstellung sterilfiltriert und in 2ml-Vials gefüllt. Die Proben wurden bei 20°C vakuumgetrocknet und nach vorgegebenen Zeiten wurden von beiden Rezepturen Proben entnommen. Bei den entnommenen Mustern wurden die Restfüllmenge, der Wassergehalt nach Karl Fischer und der Tg bestimmt. Während der ganzen Trockenzeit wurde eine Plattentemperatur von 20°C gehalten. Der Kammerdruck wurde schrittweise bis auf ca. 10⁻³ mbar abgesenkt. Das Füllgewicht der Vials nimmt bei beiden Rezepturen von 7 h auf etwa 6% des Ausgangswertes ab, d.h. die Lösungen werden sehr schnell aufkonzentriert. Bei der reinen Maltose-Rezeptur entsteht zunächst eine übersättigte Lösung, die dann in einen gummiartigen Zustand übergeht. Im weiteren Verlauf der Trocknung ist ein geringer Vorteil bei der Massenabnahme in der phenylalaninhaltigen Zubereitung gegenüber der reinen Zuckerlösung erkennbar. Nach Ende der Trocknung ist bei den Maltoseproben noch ca. 5,5 % des ursprünglichen Füllgewichts in den Vials, während bei der Maltose - Phenylalaninmischung noch ca. 4,9 % der Füllmenge in den Vials vorhanden ist.

Dieses Ergebnis wird noch deutlicher, wenn man statt der Veränderung des Füllgewichtes die Veränderung des Wassergehaltes in den Proben betrachtet. Zu Beginn der Trocknung besteht die Lösung zu 95,08% aus Wasser, d.h. pro Vial sind ca. 965 mg Wasser enthalten. Ziel ist ein trockenes Produkt mit einer Restfeuchte von 1- 2 %. Bei einer Feststoffmenge von 50 mg entsprechend diese 1 - 2% dann 0.5 - 1 mg Wasser pro Fläschchen. Dementsprechend müssen während der Trocknung ca. 99,95 % des vorhandenen Wassers aus der Probe sublimieren, um ein trockenens Produkt zu erhalten. Im fortgeschrittenen Stadium der Trocknung wurde der Wassergehalt der Proben nach Karl Fischer bestimmt. Dies erfolgte bei den phenylalaninhaltigen Proben durch direktes Einbringen der Proben in die Methanollösung. Der sehr klebrige Zucker läßt sich nicht direkt in die Methanolösung überführen. Dieser wurde zunächst in wasserfreiem DMF gelöst. Dann wurde der Wassergehalt dieser Lösung bestimmt. Abbildung 3a zeigt die Ergebnisse der so erfolgten Restwasserbestimmung. Man erkennt sehr deutlich die Überlegenheit der aminosäurehaltigen Zubereitung. Schon nach 17 h Trocknung ist hier der Restwassergehalt auf 2,7 % zurückgegangen, während er bei der Maltose-Rezeptur noch bei 13,59% liegt. Dieses Ergebnis zeigt den Vorteil der phenylalaninhaltigen Lösung. Es ist nicht möglich, Maltose unter diesen Verfahrensbedingungen innerhalb von 48 h zu trocknen. Nach Ende der Trocknung liegt bei RT immer noch ein 'rubber' mit einem beträchtlichen Restwassergehalt vor. Neben den Restwassergehalten wurden auch die Glasübergangstemperaturen der einzelnen Proben mittels DSC bestimmt. Da die Glasübergangstemperaturen direkt mit dem Wassergehalt der Proben korrespondieren, weist die Maltose-Phenylalaninmischung deutlich höhere Werte auf. Das Ergebnis zeigt, daß der Tg der Aminosäure-Zuckermischungen bereits nach ca. 10 h im Bereich der Plattentemperatur liegt. Die entsprechenden Meßwerte sind in Abb. 3 b dargestellt. Da in diesem Stadium der Trocknung nur noch sehr wenig Wasser verdampft, liegt auch die Produkttemperatur im Bereich der Plattentemperatur. So liegt bereits nach 10 h des Trocknungsprozesses in den Vials bei RT ein Glas vor. Im Falle der Stabilisierung von Proteinen mit einer solchen Rezeptur bedeutet dies, daß das Protein bereits nach 10 h in ein stabilisierendes Glas eingebettet ist. Die Zeit in der das Protein in einer aufkonzentrierten Lösung oder in einem 'rubber' verweilt ist also sehr kurz, was für die Sabilität des Wirkstoffes von großem Vorteil ist. Der reine Zucker liegt dagegen selbst nach Abschluß der Trocknng bei Raumtermperatur noch als 'rubber' vor, und hat so im Falle eines proteinhaltigen Produktes keine stabiliserende Wirkung auf den Wirkstoff.

Die verschiedenen, proteinhaltigen Zubereitungen unterscheiden sich in den physikalischen Restgrößen nicht von den wirkstoffreien Basisrezepturen.

## Patentansprüche

1. Verfahren zur Herstellung von trockenen, teilamorphen Produkten. die neben einer oder mehreren Substanzen der Gruppen Proteine, Humanpeptide, Glycoproteine. Lipoproteine, Enzyme, Coenzyme, Antikörper. Antikörperfragmente, Viren. Virusbestandteile. Zellen und Zellbestandteile, Vaccine, DNA, RNA. PNA, und deren Derivate Substanzgemische enthalten, wobei die Substanzgemische ausgewählt sind aus je mindestens einer Substanz der Gruppe
(i) Kohlenhydrat oder Zwitterion mit polarem Rest und deren Derivate. und
(ii) Zwitterion mit apolaren Rest und dessen Derivate,
**dadurch gekennzeichnet, daß** eine Lösung aus einer oder mehreren Substanzen der Gruppen Proteine, Humanpeptide. Glycoproteine, Lipoproteine, Enzyme, Coenzyme, Antikörper, Antikörperfragmente, Viren, Virusbestandteile, Zellen und Zelibestandteile, Vaccine, DNA, RNA, PNA, und deren Derivate und der Substanzen (i) und (ii) hergestellt wird und die Lösung ohne Einfrieren der Lösung getrocknet wird.

2. Verfahren nach Anspruch 1, wobei das Zwitterion mit apolarem Rest eine Aminocarbonsäure oder deren Derivat ist.

3. Verfahren nach einem der Ansprüche 1 oder 2, wobei das Zwitterion mit polarem Rest eine Aminocarbonsäure oder deren Derivat ist.

4. Verfahren nach Anspruch 1, **dadurch gekennzeichnet, daß** Substanzgemische aus einem oder mehreren Stoffen der jeweiligen Gruppe,
Mono-, Disacharide, Arginin, Asparaginsäure, Citrullin, Glutaminsäure, Ornithin, Histidin, Lysin und deren Derivate und
Acetylphenylalaninethylester, Alanin, Cystein, Glycin, Isoleucin, Leucin. Methionin, Phenylalanin, Tryptophan. Valin, Sarcosin und deren Derivate ausgewählt werden.

5. Verfahren nach einem der Ansprüche 1-5, **dadurch gekennzeichnet, daß** die Lösung zusätzlich übliche Hilfsstoffe aus den Gruppen Puffer, Tenside, Antioxidantien, Isotonisierungsmittel, Konservierungsmittel enthält.

6. Verfahren nach einem der Ansprüche 1-5, **dadurch gekennzeichnet, daß** die Trocknung mittels Vakuumtrocknung erfolgt.

7. Verfahren nach einem der Ansprüche 1-6, **dadurch gekennzeichnet, daß** die Vakuumtrocknung in kontinuierlicher Trocknungsweise erfolgt.

8. Verfahren nach einem der Ansprüche 1-5, **dadurch gekennzeichnet, daß** die Trocknung mittels Sprühtrocknung erfolgt.

9. Verfahren nach einem der Ansprüche 1-5, **dadurch gekennzeichnet, daß** die Trocknung mittels Walzentrocknung erfolgt.

10. Verfahren nach einem der Ansprüche 1-5, **dadurch gekennzeichnet, daß** die Trocknung mittels Infrarotstrahlen, Mikrowellen oder anderen Verfahren der Strahlungstrocknung erfolgt.

11. Verfahren nach einem der Ansprüche 1-10, **dadurch gekennzeichnet, daß** ein Zwitterion mit apolarem Rest derart ausgewählt wird, daß das getrocknete Substanzgemisch einen erhöhten Glaspunkt gegenüber einem Substanzgemisch ohne entsprechenden Zusatz aufweist.

12. Verfahren nach einem der Ansprüche 1-7, **dadurch gekennzeichnet, daß** die Trocknung in einer Gefriertrockenanlage ohne vorheriges Einfrieren erfolgt.

13. Verfahren nach einem der Ansprüche 1-7, 11 oder 12, **dadurch gekennzeichnet, daß** die Trocknung des Substanzgemisches in Einzeldosisbehältern durchgeführt wird.

14. Verfahren nach einem der Ansprüche 1-12, **dadurch gekennzeichnet, daß** das erhaltene Stoffgemisch anschließend zu Pulver gemahlen wird.

15. Substanzgemische enthaltend, neben einer oder mehreren Substanzen der Gruppen Proteine. Humanpeptide. Glycoproteine, Lipoproteine. Enzyme. Coenzyme, Antikörper. Antikörperfragmente. Viren, Virusbestandteile, Zellen und Zellbestandteile, Vaccine, DNA, RNA, PNA, und deren Derivate. Stoffe ausgewählt aus je einer oder mehreren Substanzen der folgenden Gruppen
(i) einem Kohlenhydrat oder Zwitterion mit polaren Resten und deren Derivate
(ii) Zwitterionen mit apolaren Resten und deren Derivate,
**dadurch gekennzeichnet, daß** die Substanzgemische durch Zusatz einer oder mehrerer Substanzen der Gruppe (ii) eine erhöhte Glasübergangstemperatur gegenüber Substanzen der Gruppe (i) ohne entsprechenden Zusatz. aufweisen, wobei Lyophilisate ausgenommen sind.

16. Substanzgemische erhältlich nach einem Verfahren, nach einem der Ansprüche 1-14.

17. Substanzgemische nach Anspruch 15 oder 16, **dadurch gekennzeichnet, daß** sie teilamorph sind.

18. Substanzgemische nach einem der Ansprüche 15-17, **dadurch gekennzeichnet, daß** die Glasübergangstemperarur über 4°C liegt, bevorzugt über 20 °C, und die Restfeuchte weniger als 6% (g/g), vorzugsweise weniger als 4% (g/g) beträgt.

19. Substanzgemische nach einem der Ansprüche 15-18. **dadurch gekennzeichnet. daß** sie eine mindestens 10% höhere scheinbare Dichte als Lyophilisate aufweisen.

20. Substanzgemische nach einem der Ansprüche 15-19, **dadurch gekennzeichnet. daß** sie eine spröde, teilamorphe, glasartige, kompakte Struktur aufweisen.

21. Substanzgemische nach einem der Ansprüche 15-20. **dadurch gekennzeichnet. daß** sie die teilamorphe Struktur über Lagerzeiten von mindestens 2 Wochen behalten.

22. Substanzgemische nach einem der Ansprüche 15-21, **dadurch gekennzeichnet. daß** deren Trockungszeiten gegenüber Substanzen der Gruppe (i) mindestens halbiert sind.

23. Diagnostische Mittel, **dadurch gekennzeichnet, daß** sie Substanzgemische nach einem der Ansprüche 15-22 enthalten.

24. Venvendung von Substanzgemischen gemäß einem der Ansprüche 15-22 zur Herstellung von Diagnostika.

25. Therapeutische Zubereitungen, **dadurch gekennzeichnet, daß** sie Stoffgemische nach einem der Ansprüche 15-22 enthalten.

26. Verwendung von Substanzgemischen gemäß einem der Ansprüche 15-22 neben üblichen Hilfs- und Zusatzstoffen zur Herstellung von therapeutischen Mitteln.

27. Verwendung von Substanzgewischen aus je mindestens einer Substanz ausgewählt aus
(i) Kohlehydrat oder Zwitterion mit polarem Rest und deren Derivate und
(ii) Zwitterion mit apolarem Rest und dessen Derivate
zur Stabilisierung von einer oder mehreren Substanzen der Gruppen Proteine. Humanpeptide, Glycoproteine. Lipoproteine. Enzyme. Coenzyme. Antikörper. Antikörperfraamente. Viren. Virusbestandteile, Zellen und Zellbestandteile. Vaccine, DNA. RNA. PNA, und deren Derivate
in einem Produkt entstanden durch Trocknen ohne Einfrieren.

## Claims

1. A process for the production of dry, partially amorphous products which contain, in addition to one or more substances from the groups proteins, human peptides, glycoproteins, lipoproteins, enzymes, coenzymes, antibodies, antibody fractions, viruses, virus components, cells and cell components, vaccines, DNA, RNA, PNA, and derivatives thereof, substance mixtures, whereby the substance mixtures are selected from in each case at least one substance from the group
(i) carbohydrate or zwitterion with a polar residue and derivatives thereof, and
(ii) zwitterion with an apolar residue and derivatives thereof,
**characterized in that** a solution of one or more substances from the groups proteins, human peptides, glycoproteins, lipoproteins, enzymes, coenzymes, antibodies, antibody fractions, viruses, virus components, cells and cell components, vaccines, DNA, RNA, PNA, and derivatives thereof and of the substances (i) and (ii) is prepared and the solution is dried without freezing the solution.

2. A process according to claim 1, wherein the zwitterion with an apolar residue is an aminocarboxylic add or derivative thereof.

3. A process according to one of claims 1 or 2, wherein the zwitterion with a polar residue is an aminocarboxylic add or derivative thereof.

4. A process according to claim 1, **characterized in that** the substance mixtures are selected from one or more substances of the respective group,
monosaccharides, disaccharides, arginine, aspartic acid, citrulline, glutamic acid, ornithine, hstidine, lysine and derivatives thereof and
acetylphenylalanine ethyl ester, alanine, cysteine, glycine, isoleudne, leucine, methionine, phenylalanine, tryptophan, valine, sarcosine and derivatives thereof.

5. A process according to any one of claims 1-5, **characterized in that** the solution additionally contains common auxiliary substances from the groups buffers, tensides, antioxidants, isotonizing agents, preservatives.

6. A process according to any one of claims 1-5, **characterized in that** the drying is effected by means of vacuum drying.

7. A process according to any one of claims 1-6, **characterized in that** the vacuum drying is effected in a continuous drying procedure.

8. A process according to any one of claims 1-5, **characterized in that** the drying is effected by means of spray drying.

9. A process according to any one of claims 1-5, **characterized in that** the drying is effected by means of drum drying.

10. A process according to any one of claims 1-5, **characterized in that** the drying is effected by means of infrared rays, microwaves or other methods of radiation drying.

11. A process according to any one of claims 1-10, **characterized in that** a zwitterion with an apolar residue is selected such that the dried substance mixture has an increased glass point compared with a substance mixture without a corresponding addition.

12. A process according to any one of claims 1-7, **characterized in that** the drying is effected in a freeze-drying apparatus without prior freezing.

13. A process according to any one of claims 1-7, 11 or 12, **characterized in that** the drying of the substance mixture is carried out in single dosage containers.

14. A process according to any one of claims 1-12, **characterized in that** the substance mixture obtained is subsequently ground to a powder.

15. Substance mixtures containing, in addition to one or more substances from the groups proteins, human peptides, glycoproteins, lipoproteins, enzymes, coenzymes, antibodies, antibody fractions, viruses, virus components, cells and cell components, vaccines, DNA, RNA, PNA, and derivatives thereof, substances selected from in each case one or more substances from the following groups
(i) a carbohydrate or zwitterion with polar residues and derivatives thereof
(ii) zwitterion with apolar residues and derivatives thereof,
**characterized in that** the substance mixtures have by the addition of one or more substances of group (ii) an increased glass transition temperature compared with substances of group (i) without the corresponding addition, whereby lyophilizates are excluded.

16. Substance mixtures obtainable according to a process according to any one of claims 1-14.

17. Substance mixtures according to claim 15 or 16, **characterized in that** they are partially amorphous.

18. Substance mixtures according to any one of claims 15-17, **characterized in that** the glass transition temperature lies above 4°C, preferably above 20°C, and the residual moisture is less than 6% (g/g), preferably less than 4% (g/g).

19. Substance mixtures according to any one of claims 15-18, **characterized in that** they have an at least 10% higher apparent density than lyophilizates,

20. Substance mixtures according to any one of claims 15-19, **characterized in that** they have a brittle, partially amorphous, glass-like, compact structure.

21. Substance mixtures according to any one of claims 15-20, **characterized in that** they retain the partially amorphous structure over storage periods of at least 2 weeks.

22. Substance mixtures according to any one of claims 15-21, **characterized in that** the drying periods are at least halved compared with substances of group (i).

23. Diagnostic agents, **characterized in that** they contain substance mixtures according to any one of claims 15-22.

24. The use of substance mixtures in accordance with any one of claims 15-22 for the production of diagnostic agents.

25. Therapeutic preparations, **characterized in that** they contain substance mixtures according to anyone of claims 15-22.

26. The use of substance mixtures in accordance with any one of claims 15-22 together with common auxiliary substances and additives for the production of therapeutic agents.

27. The use of substance mixtures from in each case at least one substance selected from
(i) carbohydrate or zwitterion with a polar residue and derivatives thereof, and
(ii) zwitterion with an apolar residue and derivatives thereof
for the stabilization of one or more substances from the groups proteins, human peptides, glycoproteins, lipoproteins, enzymes, coenzymes, antibodies, antibody fractions, viruses, virus components, cells and cell components, vaccines, DNA, RNA, PNA, and derivatives thereof, in a product produced by drying without freezing.

## Revendications

1. Procédé pour la préparation de produits secs partiellement amorphes qui, outre une ou plusieurs substances appartenant aux groupes des protéines, peptides humains, glycoprotéines, lipoprotéines, enzymes, coenzymes, anticorps, fragments d'anticorps, virus, composants de virus, cellules et composants cellulaires, vaccins, ADN, ARN, APN et leurs dérivés, contiennent des mélanges de substances, les mélanges de substances étant choisis parmi au moins une substance de chacun des groupes suivants
(I) un glucide ou un zwitterion à reste polaire et leurs dérivés,
(II) un zwitterion à reste non polaire et ses dérivés,
**caractérisé en ce qu'**on prépare une solution à partir d'une ou plusieurs substances appartenant aux groupes des protéines, peptides humains, glyco-protéines, lipoprotéines, enzymes, coenzymes, anticorps, fragments d'anticorps, virus, composants de virus, cellules et composants cellulaires, vaccins, ADN, ARN, APN et leurs dérivés, et des substances (I) et (II), et on sèche la solution sans congélation de la solution,

2. Procédé selon la revendication 1, dans lequel le zwitterion à reste non polaire est un acide carboxylique aminé ou un dérivé de celui-ci.

3. Procédé selon la revendication 1 ou 2, dans lequel le zwitterion à reste polaire est un acide carboxylique aminé ou un dérivé de celui-ci.

4. Procédé selon la revendication 1, **caractérisé en ce qu'**on choisit des mélanges de substances parmi une ou plusieurs substances de chaque groupe, monosaccharides, disaccharides, arginine, acide aspartique, citrulline, acide glutamique, ornithine, histidine, lysine et leurs dérivés et
ester éthylique d'acétylphénylalanine, alanine, cystéine, glycine, isoleucine, leucine, méthionine, phénylalanine, tryptophane, valine, sarcosine et leurs dérivés.

5. Procédé selon l'une quelconque des revendications 1 à 5, **caractérisé en ce que** la solution contient en outre des adjuvants usuels appartenant aux groupes des tampons, tensioactifs, antioxydants, agents d'isotonie, conservateurs.

6. Procédé selon l'une quelconque des revendications 1 à 5, **caractérisé en ce que** le séchage s'effectue par séchage sous vide.

7. Procédé selon l'une quelconque des revendications 1 à 6, **caractérisé en ce que** le séchage sous vide s'effectue en un mode de séchage continu.

8. Procédé selon l'une quelconque des revendications 1 à 5, **caractérisé en ce que** le séchage s'effectue par séchage par atomisation.

9. Procédé selon l'une quelconque des revendications 1 à 5, **caractérisé en ce que** le séchage s'effectue par séchage sur un sécheur à cylindres.

10. Procédé selon l'une quelconque des revendications 1 à 5, **caractérisé en ce que** le séchage s'effectue au moyen de rayons infrarouges, de micro-ondes ou d'autres procédés de séchage par irradiation.

11. Procédé selon l'une quelconque des revendications 1 à 10, caractérlsé en ce qu'on choisit un zwitterion à reste non polaire de sorte que le mélange de substances séché présente un point de transition vitreuse augmenté par rapport à un mélange de substances sans addition correspondante.

12. Procédé selon l'une quelconque des revendications 1 à 7, **caractérisé en ce que** le séchage s'effectue dans une installation de lyophilisation sans congélation préliminaire.

13. Procédé selon l'une quelconque des revendications 1 à 7, 11 et 12,
**caractérisé en ce que** le séchage du mélange de substances s'effectue en récipients pour doses unitaires.

14. Procédé selon l'une quelconque des revendications 1 à 12, **caractérisé en ce que** le mélange de substances est ensuite broyé en une poudre.

15. Mélanges de substances contenant, outre une ou plusieurs substances appartenant aux groupes des protéines, peptides humains, glycoprotéines, lipoprotéines, enzymes, coenzymes, anticorps, fragments d'anticorps, virus, composants de virus, cellules et composants cellulaires, vaccins, ADN, ARN, APN et leurs dérivés, des substances choisies parmi une ou plusieurs substances de chacun des groupes suivants
(I) un glucide ou un zwitterion à reste polaire et leurs dérivés,
(II) des zwitterions à restes non polaires et leurs dérivés,
**caractérisés en ce que** les mélanges de substances présentent, grâce à l'addition d'une ou plusieurs substances du groupe (II), une température de transition vitreuse plus élevée par rapport à celle des substances du groupe (I) sans addition correspondante,
à l'exclusion des lyophilisats.

16. Mélanges de substances pouvant être obtenus par un procédé selon l'une quelconque des revendications 1 à 14.

17. Mélanges de substances selon la revendication 15 ou 16, **caractérisés en ce qu'**ils sont partiellement amorphes.

18. Mélanges de substances selon l'une quelconque des revendications 15 à 17, **caractérisés en ce que** la température de transition vitreuse est supérieure à 4°C, de préférence, supérieure à 20°C, et l'humidité résiduelle est inférieure à 6 % (p/p), de préférence, inférieure à 4 % (p/p).

19. Mélanges de substances selon l'une quelconque des revendications 15 à 18, **caractérisés en ce qu'**ils présentent une densité apparente supérleure d'au moins 10 % à celle de lyophilisats.

20. Mélanges de substances selon l'une quelconque des revendications 15 à 19, **caractérisés en ce qu'**ils présentent une structure compacte, vitreuse, partiellement amorphe, cassante.

21. Mélanges de substances selon l'une quelconque des revendications 15 à 20, **caractérisés en ce qu'**ils conservent la structure partiellement amorphe pendant des périodes de stockage d'au moins 2 semaines.

22. Mélanges de substances selon l'une quelconque des revendications 15 à 21, **caractérisés en ce que** leurs temps de séchage sont au moins réduits de moitié par rapport aux substances du groupe (I).

23. Produits de diagnostic, **caractérisés en ce qu'**ils contiennent des mélanges de substances selon l'une quelconque des revendications 15 à 22.

24. Utilisation des mélanges de substances selon l'une quelconque des revendications 15 à 22, pour la préparation de produits de diagnostic.

25. Préparations thérapeutiques, **caractérisées en ce qu'**elles contiennent des mélanges de substances selon l'une quelconque des revendications 15 à 22.

26. Utilisation des mélanges de substances selon l'une quelconque des revendications 15 à 22, en plus d'adjuvants et additifs usuels, pour la préparation de compositions thérapeutiques.

27. Utilisation de mélanges de substances à base d'au moins une substance choisie dans chacun des groupes suivants :
(I) un glucide ou un zwitterion à reste polaire et leurs dérivés et
(II) un zwitterion à reste non polaire et ses dérivés,
pour la stabilisation d'une ou plusieurs substances appartenant aux groupes des protéines, peptides humains, glycoprotéines, lipoprotéines, enzymes, coenzymes, anticorps, fragments d'anticorps, virus, composants de virus, cellules et composants cellulaires, vaccins, ADN, ARN, APN et leurs dérivés, dans un produit obtenu par séchage ou congélation.
